# EUROPEAN PATENT SPECIFICATION

(11) **EP 2 970 922 B1**
(45) Date of publication and mention of the grant of the patent: **13.12.2017**
(21) Application number: 14762285.6
(22) Date of filing: 15.03.2014
(51) Int. Cl.: C12N 9/00, C12Q 1/68, C12P 19/34

(54) **THERMOSTABLE BLUNT-END LIGASE AND METHODS OF USE**
WÄRMESTABILE OFFENENDIGE LIGASE UND VERFAHREN ZUR VERWENDUNG
LIGASE À EXTRÉMITÉ FRANCHE THERMOSTABLE ET PROCÉDÉS D'UTILISATION

(30) Priority: 15.03.2013 US 201361802124 P
(43) Date of publication of application: 20.01.2016
(73) Proprietor: Theranos, Inc., Palo Alto, CA 94304 (US)
(72) Inventor: CHRISTIANS, Frederick, Palo Alto, CA 94304 (US)
(74) Representative: Avidity IP
(86) International application number: PCT/US2014/030003
(87) International publication number: WO 2014/145269

(56) References cited:
- WO-A1-94/02615
- WO-A1-2011/034449
- WO-A1-2011/034449
- WO-A1-2015/076919
- US-B2- 7 208 278
- G Lauer ET AL: "Cloning, nucleotide sequence, and engineered expression of Thermus thermophilus DNA ligase, a homolog of Escherichia coli DNA ligase", Journal of Bacteriology, 1 August 1991 (1991-08-01), pages 5047-5053, XP055284088, UNITED STATES Retrieved from the Internet: URL:http://jb.asm.org/content/173/16/5047. full.pdf#page=1&view=FitH [retrieved on 2016-08-26]
- LAUER GAIL ET AL.: 'Cloning, Nucleotide Sequence, and Engineered Expression of Thermus thermophilus DNA Ligase, a Homolog of Escherichia coli DNA Ligase.' JOURNAL OF BACTERIOLOGY vol. 173, no. 16, August 1991, pages 5047 - 5053, XP055284088
- DATABASE UNIPROTKB [Online] 05 September 2012 XP008182953 Retrieved from UNIPROT Database accession no. 13RFS7

## Description

### BACKGROUND

A variety of methods for the amplification of nucleic acids are known. For example, polymerase chain reaction ("PCR") (see, e.g. U.S. Patent No. 4,683,202) is a popular method for the amplification of nucleic acids. To successfully perform a PCR reaction, the reaction must be performed at multiple different temperatures. This requires hardware or other mechanisms for repeatedly changing the temperature of the PCR reaction. Another method for amplification of nucleic acids is referred to as loop-mediated isothermal amplification ("LAMP") (see, e.g. U.S. Patent No. 6,410,278). LAMP reactions may be performed isothermally, but typically involve the use of four different primers which recognize a total of six distinct sequences on the target nucleic acid.

Some amplification methods utilize DNA ligation. DNA ligation is a common molecular biology method for joining multiple DNA fragments. Ligases can seal single-strand nicks in duplex DNA, join two pieces with complementary "sticky" ends, and in some cases join two pieces with blunt, non-sticky ends. These methods find use in molecular cloning, nucleic acid diagnostics/detection, nucleic acid amplification, and related activities. Ligases and ligation methods are described, for example, in U.S. Patent Application Publication No. 20120214208; U.S. Patent 7,927,853; de Lumley et al., J. Mol. Biol. (2004) 339, 1059-1075; and Rolland et al., FEMS Microbiol. Lett. (2004) 236, 267-273.

Ligation of two strands of "sticky-ended" DNA is possible, where the substrates to be joined are already pre-positioned in the case of single-strand nick sealing, and the affinity of sticky ends for each other helps drive sticky-end ligation. However, since DNA ligation depends upon the juxtaposition of the two substrates to be joined, DNA ligation is more difficult in situations where the juxtaposition of the two substrates is more difficult, or the strands are likely to become mis-aligned or to separate readily. For this reason, blunt-end and high temperature ligations are two types of ligation that are particularly difficult. Blunt-end ligation, however, depends upon random interactions of the substrates. Two adjustments in blunt-end ligation are commonly made to drive the substrate interactions: low temperature to slow down molecular motion so that random interactions last longer and thus give the ligase a better chance to join the fragments; and molecular crowding reagents such as polyethylene glycol to increase the local concentrations of substrates. Likewise, high temperature ligations are challenging because the interactions of DNA substrates are briefer. The least efficient case is thus a high temperature blunt-end ligation in which molecular crowding reagents cannot be used.

A thermostable ligase, T4 DNA ligase, has been described that can perform blunt-end ligations; however, it is inactivated at temperature above approximately 45°C, so that the range of temperature at which T4 DNA ligase is stable is relatively small. A few other examples of ligases that can be induced to join blunt-ended fragments are known, but these ligases appear to do so only in the presence of high concentrations of molecular crowding agents such as 50% polyethylene glycol (PEG). However, the utility of ligases that require such molecular crowding agents is unclear, since 50% PEG which DNA polymerization (which is required for DNA amplification).WO2011/034449 discloses fusion proteins between DNA binding domains and DNA-ligases able to perform blunt-end ligations. An additional N-terminal his-tag is present on the fusion proteins. The presence of a spacer or linker between the domains is also foreseen in the description. The resulting fusion proteins have an increased blunt-end ligation activity, tested at temperatures below 60°C.

Accordingly, in order to facilitate the generation of amplified nucleic acids for the many and growing number of applications which use amplified nucleic acids, new methods and reagents for the amplification of nucleic acids are desired.

### SUMMARY

Fusion proteins having thermostable blunt-end ligase activity are provided. Such thermostable blunt-end ligases are useful for DNA amplification, sequencing, production of recombinant DNA (e.g., as a result of such blunt-end fusions) and recombinant fusion proteins (e.g., proteins encoded by recombinant DNA produced as a result of such blunt-end fusions), and for other purposes. Many molecular biology schemes involve ligases; such schemes would benefit from the ability to perform such schemes at elevated temperatures. Thermostable blunt-end DNA ligases disclosed herein are suitable for use in ligation schemes which would benefit from at least one elevated temperature incubation, such as an incubation at about 60-65°C or higher, including incubation schemes at temperatures as high as about 94°C. A thermostable blunt-end ligase as disclosed herein would be useful for such schemes.

In addition, thermostable blunt-end ligases as disclosed herein may enable high temperature blunt-end ligation without the need for molecular crowding agents. Accordingly, thermostable blunt-end ligase as disclosed herein may be useful for many nucleic acid ligation-amplification schemes, including nucleic acid ligation-amplification schemes which operate at a uniform temperature (e.g., at about 60°C or higher), and including nucleic acid ligation-amplification schemes which require temperature cycling such as cycling from, e.g., about 94°C to about 60°C (or higher) for one, two, three, or more cycles. Nucleic acid ligation-amplification schemes which may benefit from the use of thermostable blunt-end DNA ligases disclosed herein may operate at other temperatures as well, e.g., depending on the temperature resistance characteristics of the novel fusion proteins disclosed herein and the requirements of the difference nucleic acid ligation-amplification schemes. Thermostable blunt-end DNA ligases disclosed herein provide the ability to use high temperatures in nucleic acid ligation-amplification schemes and thereby enable higher specificity target amplification, for example, by permitting temperature denaturation of double-stranded DNA templates as well as specific primer binding.

Accordingly, Applicant discloses a thermostable blunt-end nucleic acid ligase comprising a fusion protein comprising a nucleic acid ligase and a nucleic acid binding protein, wherein said thermostable blunt-end nucleic acid ligase is suitable for use in a blunt-ended nucleic acid ligation reaction performed at an elevated temperature. In embodiments, an elevated temperature comprises a temperature of about 60 °C or higher. In embodiments, an elevated temperature comprises a temperature of about 65 °C, or about 70 °C, or about 75 °C, or about 80 °C, or about 85 °C, or about 90 °C, or about 95 °C, or higher. Applicant further discloses an article of manufacture, comprising such a thermostable blunt-end nucleic acid ligase, and a container. In embodiments, the article of manufacture further comprises a buffer. Applicant further discloses a method of ligating blunt-end nucleic acids at an elevated temperature, comprising using a thermostable blunt-end nucleic acid ligase as disclosed herein, wherein the use may be at a temperature of about 60 °C or higher. Applicant further discloses a device for analyzing a sample containing nucleic acids, comprising a thermostable blunt-end nucleic acid ligase comprising a fusion protein comprising a nucleic acid ligase and a nucleic acid binding protein, wherein said thermostable blunt-end nucleic acid ligase is suitable for use in a blunt-ended nucleic acid ligation reaction performed at about 60 °C or higher.

In embodiments, Applicant discloses herein a thermostable blunt-end DNA ligase comprising a fusion protein comprising a DNA ligase and a DNA binding protein, wherein said thermostable blunt-end DNA ligase is suitable for use in a blunt-ended DNA ligation reaction performed at an elevated temperature, for example,, at a temperature of about 60 °C or higher. In embodiments, the thermostable blunt-end DNA ligase comprises a T4 DNA ligase with an N-terminal p50 fusion.

In a particular embodiment, the thermostable blunt-end DNA ligase comprises the amino acid sequence (SEQ ID NO: 1):

mghhhhhhhhhhssghiegrasadgpylqileqpkqrgfrfryvcegpshgglpgasseknkksypqvkicny
vgpakvivqlvtngknihlhahslvgkhcedgictvtagpkdmvvgfanlgilhvtkkkvfetlearmteacirgy
npgllvhpdlaylqaegggdrqlgdrekelirqaalqqtkemdlsvvrlmftaflpdstgsftrrlepvvsdaiydsk
apnasnlkivrmdrtagcvtggeeiyllcdkvqkddiqirfyeeeenggvwegfgdfsptdvhrqfaivfktpkyk
dinitkpasvfvqlrrksdletsepkpflyypeikdkeevqrkrqkgssgtsgggsgggmtleearkrvnelrdliry
hnyryyvladpeisdaeydrllrelkeleerfpelkspdsptlqvgarpleatfrpvrhptrmysldnafnldelkafe
erieralgrkgpfaytvehkvdglsvnlyyeegvlvygatrgdgevgeevtqnlltiptiprrlkgvperlevrgevy
mpieaflrlneeleergerifknprnaaagslrqkdpritakrglratfyalglgleeveregvatqfallhwlkekgfp
vehgyaravgaegveavyqdwlkkrralpfeadgvvvkldelalwrelgytaraprfaiaykfpaeeketrlldvv
fqvgrtgrvtpvgilepvflegsevsrvtlhnesyieeldirigdwvlvhkaggvipevlrvlkerrtgeerpirwpet
cpecghrllkegkvhrcpnplcpakrfeairhfasrkamdiqglgeklierllekglvkdvadlyrlrkedlvglerm
geksaqnllrqieeskkrglerllyalglpgvgevlarnlaarfgnmdrlleasleelleveevgeltarailetlkdpafr
dlvrrlkeagvemeakekggealkgltfvitgelsrpreevkallrrlgakvtdsvsrktsylvvgenpgsklekaral
gvptlteeelyrlleartgkkaeelv.

A thermostable blunt-end DNA ligase as disclosed herein may comprise a fusion protein which comprises a component selected from a peptide linker, an N-terminal addition, a C-terminal addition, a tag peptide, a D-amino acid, and a peptide mimetic. In embodiments, a thermostable blunt-end DNA ligase as disclosed herein comprises a fusion protein which comprises a component selected from a sugar and a polymer.

In embodiments, a thermostable blunt-end DNA ligase as disclosed herein is suitable for use in a blunt-ended DNA ligation reaction performed at about 75 °C.

In embodiments, a thermostable blunt-end DNA ligase as disclosed herein is capable of making concatamers upon multiple ligation events in a blunt-ended DNA ligation reaction performed at about 60 °C or higher.

In embodiments, the thermostable blunt-end DNA ligase as disclosed herein is suitable for use in a nucleic acid amplification scheme which operates at a uniform temperature of about 60 °C or higher.

In embodiments, the thermostable blunt-end DNA ligase as disclosed herein is suitable for use in a nucleic acid amplification scheme which comprises temperature cycling. In embodiments, such temperature cycling comprises temperature cycling to temperatures of about 60 °C or higher. In embodiments, such temperature cycling comprises temperature cycling from about 94 °C to about 60 °C. In embodiments, such temperature cycling comprises two or more cycles of temperature cycling, and, in embodiments, comprises three or more cycles of temperature cycling.

Applicant further discloses a method of ligating blunt-end DNA at elevated temperature, comprising using a thermostable blunt-end DNA ligase as disclosed herein, wherein the use may be at an elevated temperature. In embodiments, an elevated temperature comprises a temperature of about 60 °C or higher. In embodiments, an elevated temperature comprises a temperature of about 75 °C; and, in further embodiments, may comprise a temperature higher than about 75 °C.

Applicant discloses herein an article of manufacture, comprising a thermostable blunt-end DNA ligase and a container, wherein the thermostable blunt-end DNA ligase is a thermostable blunt-end DNA ligase as disclosed herein. In embodiments, the article of manufacture further comprises a buffer.

Applicant discloses herein a device for analyzing a sample containing DNA, comprising a thermostable blunt-end DNA ligase, wherein the thermostable blunt-end DNA ligase is a thermostable blunt-end DNA ligase as disclosed herein.

### BRIEF DESCRIPTION OF THE FIGURE

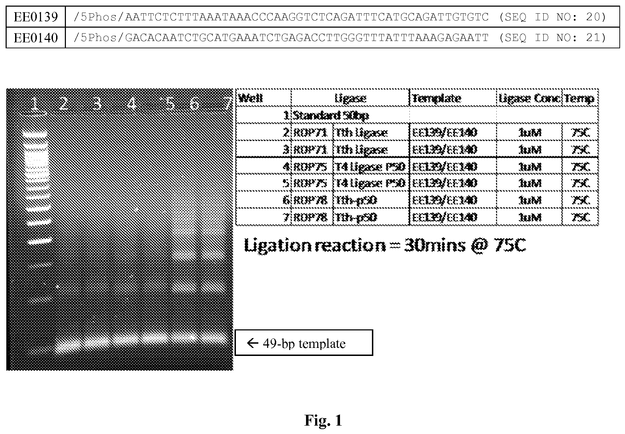

Fig. 1 shows an example of the results of a blunt-ended ligation reaction performed at 75 °C using a thermostable, blunt-end DNA ligase as provided herein. The DNA substrate was a 49-bp duplex DNA made by annealing oligonucleotides EE0139 (SEQ ID NO:20) and EE0140 (SEQ ID NO: 21).

### DETAILED DESCRIPTION

Thermostable, blunt-end ligases are provided herein. As disclosed herein, a thermostable, blunt-end ligase may be prepared by the fusion of a DNA-binding protein to a thermostable ligase. Such ligases produced by these fusions have features and capabilities not provided by their parent compounds alone; the different portions of these fusion proteins provide activities which, when combined, provide new capabilities and unexpectedly improved activity. The DNA-binding protein portion of such fusion proteins is effective to increase the affinity of the ligase to DNA substrates, resulting in enhanced ligation in the challenging conditions of high temperature, blunt-end ligation. The DNA ligase portion surprisingly retains its ability to ligate DNA when combined with a foreign protein (the DNA-binding protein portion). Together, the combined portions unite in novel fusion proteins that are able to ligate blunt-ended DNA substrates even at high temperatures, providing increased ligation activity at high temperatures unavailable by the use of the original, unmodified ligases.

### DEFINITIONS

Before the present novel ligases and ligation methods are disclosed and described, it is to be understood that the terminology used herein is for the purpose of describing particular embodiments only and is not intended to be limiting. It is also to be understood that the present disclosure provides explanatory and exemplary descriptions and examples, so that, unless otherwise indicated, the molecules, compositions, assays, methods, and kits disclosed herein are not limited to the specific embodiments described herein.

It must be noted that, as used in the specification and the appended claims, the singular forms "a," "an" and "the" include plural referents unless the context clearly dictates otherwise. Thus, for example, reference to "a salt" refers to a single salt or mixtures of different salts, and the like.

As used in the description herein and throughout the claims that follow, the meaning of "in" includes "in" and "on" unless the context clearly dictates otherwise.

As used in the description herein and throughout the claims that follow, the meaning of "or" includes both the conjunctive and disjunctive unless the context expressly dictates otherwise. Thus, the term "or" includes "and/or" unless the context expressly dictates otherwise.

In this specification and in the claims that follow, reference will be made to a number of terms, which shall be defined to have the following meanings:

The term "moiety" as used herein refers to any particular composition of matter, e.g., a molecular fragment, an intact molecule, or a mixture of materials.

The term "nucleic acid" refers to nucleotides and nucleosides which make up, for example, deoxyribonucleic acid (DNA) macromolecules and ribonucleic acid (RNA) macromolecules. Nucleic acids may be identified by the base attached to the sugar (e.g., deoxyribose or ribose); as used herein, the following abbreviations for these bases are used to represent nucleic acids in sequence listings identifying and describing their structures (either upper-case or lower-case may be used).

**TABLE 1A**

| Base (in Nucleic Acid) | Letter Code |
|---|---|
| Adenine | A |
| Thymine | T |
| Guanine | G |
| Cytosine | C |
| Uracil | U |

As used herein, a "polynucleotide" refers to a polymeric chain containing two or more nucleotides. "Polynucleotides" includes primers, oligonucleotides, nucleic acid strands, etc. A polynucleotide may contain standard or non-standard nucleotides. Typically, a polynucleotide contains a 5' phosphate at one terminus ("5' terminus") and a 3' hydroxyl group at the other terminus ("3' terminus) of the chain. The most 5' nucleotide of a polynucleotide may be referred to herein as the "5' terminal nucleotide" of the polynucleotide. The most 3' nucleotide of a polynucleotide may be referred to herein as the "3' terminal nucleotide" of the polynucleotide.

The term "downstream" as used herein in the context of a polynucleotide containing a 5' terminal nucleotide and a 3' terminal nucleotide refers to a position in the polynucleotide which is closer to the 3' terminal nucleotide than a reference position in the polynucleotide. For example, in a primer having the sequence: 5' ATAAGC 3', the "G" is downstream from the "T" and all of the "A"s.

The term "upstream" as used herein in the context of a polynucleotide containing a 5' terminal nucleotide and a 3' terminal nucleotide, refers to a position in the polynucleotide which is closer to the 5' terminal nucleotide than a reference position in the polynucleotide. For example, in a primer having the sequence: 5' ATAAGC 3', the "T" is upstream from the "G", the "C", and the two "A"s closest to the "G".

As used herein, "nucleic acid" includes both DNA and RNA, including DNA and RNA containing non-standard nucleotides. A "nucleic acid" contains at least one polynucleotide (a "nucleic acid strand"). A "nucleic acid" may be single-stranded or double-stranded.

As used herein, a nucleic acid molecule which is described as containing the "sequence" of a template or other nucleic acid may also be considered to contain the template or other nucleic acid itself (e.g. a molecule which is described as containing the sequence of a template may also be described as containing the template), unless the context clearly dictates otherwise.

As used herein, a "target" nucleic acid or molecule refers to a nucleic acid of interest. A target nucleic acid / molecule may be of any type, including single-stranded or double stranded DNA or RNA (e.g. mRNA).

As used herein, "complementary" sequences refer to two nucleotide sequences which, when aligned anti-parallel to each other, contain multiple individual nucleotide bases which pair with each other. It is not necessary for every nucleotide base in two sequences to pair with each other for sequences to be considered "complementary". Sequences may be considered complementary, for example, if at least 30%, 40%, 50%, 55%, 60%, 65%, 70%, 75%, 80%, 85%, 90%, 95%, 98%, 99%, or 100% of the nucleotide bases in two sequences pair with each other. In addition, sequences may still be considered "complementary" when the total lengths of the two sequences are significantly different from each other. For example, a primer of 15 nucleotides may be considered "complementary" to a longer polynucleotide containing hundreds of nucleotides if multiple individual nucleotide bases of the primer pair with nucleotide bases in the longer polynucleotide when the primer is aligned anti-parallel to a particular region of the longer polynucleotide.

As used herein, the term "isolated" as applied to proteins, nucleic acids, or other biomolecules refers to a molecule that has been purified or separated from a component of its naturally-occurring environment (e.g. a protein purified from a cell in which it was naturally produced). An "isolated" molecule may be in contact with other molecules (for example, as part of a reaction mixture). As used herein, "isolated" molecules also include recombinantly-produced proteins or nucleic acids which have an amino acid or nucleotide sequence which occurs naturally. "Isolated" nucleic acids include polypeptide-encoding nucleic acid molecules contained in cells that ordinarily express the polypeptide where, for example, the nucleic acid molecule is at a chromosomal location different from that of natural cells. In some embodiments, "isolated" polypeptides are purified to at least 50 %, 60%, 70%, 80%, 90%, 95%, 98%, or 100% homogeneity as evidenced by SDS-PAGE of the polypeptides followed by Coomassie blue, silver, or other protein staining method.

The terms "polypeptide" and "protein" may be used interchangeably to refer to molecules comprised of amino acids linked by peptide bonds. Individual amino acids may be termed "residues" of a polypeptide or protein. The amino acid sequences of polypeptides disclosed herein may be identified by SEQ ID NO: presented as a string of letters, where the letters have the following meanings:

**TABLE 1B**

| Amino Acid | 3-Letter Code | 1-Letter Code |
|---|---|---|
| Alanine | Ala | A |
| Arginine | Arg | R |
| Asparagine | Asn | N |
| Aspartic acid | Asp | D |
| Cysteine | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

Naturally occurring amino acid residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

"Identical" or "identity," as used herein in the context of two or more polypeptide or polynucleotide sequences, can mean that the sequences have a specified percentage of residues that are the same over a specified region. The percentage can be calculated by optimally aligning the two sequences, comparing the two sequences over the specified region, determining the number of positions at which the identical residue occurs in both sequences to yield the number of matched positions, dividing the number of matched positions by the total number of positions in the specified region, and multiplying the result by 100 to yield the percentage of sequence identity. In cases where the two sequences are of different lengths or the alignment produces one or more staggered ends and the specified region of comparison includes only a single sequence, the residues of the single sequence are included in the denominator but not the numerator of the calculation.

"Homology" or "homologous" as used herein in the context of two or more polypeptide or polynucleotide sequences, can mean that the sequences have a specified percentage of residues that are either i) the same, or ii) conservative substitutions of the same residue, over a specified region. Conservative substitutions include substitutions of one amino acid by an amino acid of the same group, and include substitutions of one amino acid by an amino acid identified in Table 1C as an exemplary or as a preferred substitution. In determining homology of two sequences, identical residues and homologous residues are given equal weight. The percentage can be calculated by optimally aligning the two sequences, comparing the two sequences over the specified region, determining the number of positions at which either identical or homologous residues occur in both sequences to yield the number of matched positions, dividing the number of matched positions by the total number of positions in the specified region, and multiplying the result by 100 to yield the percentage of sequence homology. In cases where the two sequences are of different lengths or the alignment produces one or more staggered ends and the specified region of comparison includes only a single sequence, the residues of the single sequence are included in the denominator but not the numerator of the calculation.

Variant or modified polypeptides may be prepared by substituting one or more amino acid residues by a different one or more amino acid residues in the amino acid sequence of the polypeptide. Conservative substitutions will entail exchanging one member of a group for another member of the same group; such changes tend not to significantly affect the function of a polypeptide. Non-conservative substitutions will entail exchanging a member of one of these classes for another group. Substantial modifications of polypeptides may be accomplished without significantly affecting the functional characteristics of a polypeptide by selecting substitutions that maintain one or more of (a) the structure of the polypeptide backbone in the area of the substitution, for example, as a sheet or helical conformation, (b) the charge or hydrophobicity of the molecule at the target site, or (c) the bulk of the side chain.

In particular embodiments, conservative substitutions of interest are shown in Table 1C under the headings of "exemplary substitutions" and "preferred substitutions." Such conservative substitutions may not result in significant changes in biological activity.

**TABLE 1C**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Further substitutions may be performed, and suitable substitutions may be identified by scanning amino acid analysis, in which a single amino acid, or a very few amino acids, of an initial sequence are replaced. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main chain conformation of the variant [Cunningham and Wells, Science, 244: 1081-1085 (1989)].

In addition, one or more amino acids in a sequence may be substituted by a non-conventional amino acid. Conservative replacements comprising non-conventional amino acids substitute the original amino acid with a non-conventional amino acid that resembles the original in one or more of its, characteristic properties (e.g., charge, hydrophobicity, stearic bulk; for example, one may replace Val with Nval). The term "non-conventional amino acid" refers to amino acids other than conventional amino acids, and includes, for example, isomers and modifications of the conventional amino acids (e.g., D-amino acids), non-protein amino acids, post-translationally modified amino acids, enzymatically modified amino acids, constructs or structures designed to mimic amino acids (e.g., .alpha.,.alpha.-disubstituted amino acids, N-alkyl amino acids, lactic acid, .beta.-alanine, naphthylalanine, 3-pyridylalanine, 4-hydroxyproline, O-phosphoserine, N-acetylserine, N-formylmethionine, 3-methylhistidine, 5-hydroxylysine, and norleucine), and peptides having the naturally occurring amide --CONH-- linkage replaced at one or more sites within the peptide backbone with a non-conventional linkage such as N-substituted amide, ester, thioamide, retropeptide (--NHCO--), retrothioamide (--NHCS--), sulfonamido (--SO.sub.2 NH--), and/or peptoid (N-substituted glycine) linkages.

Homologous proteins and other protein variants may be provided substitution, insertion, deletion, or addition. Such substitution, insertion, deletion, or addition may include substitution, insertion, deletion, or addition of a single residue; may include substitution, insertion, deletion, or addition of two residues; and may include substitution, insertion, deletion, or addition of three, or of more, residues. For example, substitution may include replacing one, two, three, four, five, or more amino acid residues by a different residue. Substitution may be, or may include, conservative substitution. For example, insertion may include inserting one, two, three, four, five, or more amino acid residues into an amino acid sequence. For example, deletion may include deleting one, two, three, four, five, or more amino acid residues from an amino acid sequence. For example, addition may include adding one, two, three, four, five, or more amino acid residues at the N-terminal end, at the C-terminal end, or both, of an amino acid sequence.

A composition may include a buffer. Buffers include, without limitation, phosphate, citrate, ammonium, acetate, carbonate, tris(hydroxymethyl)aminomethane (TRIS), 3-(N-morpholino) propanesulfonic acid (MOPS), 3-morpholino-2-hydroxypropanesulfonic acid (MOPSO), 2-(*N*-morpholino)ethanesulfonic acid (MES), N-(2-Acetamido)-iminodiacetic acid (ADA), piperazine-N,N'-bis(2-ethanesulfonic acid) (PIPES), N-(2-Acetamido)-2-aminoethanesulfonic acid (ACES), cholamine chloride, N,N-Bis(2-hydroxyethyl)-2-aminoethanesulfonic acid (BES), 2-[[1,3-dihydroxy-2-(hydroxymethyl)propan-2-yl]amino]ethanesulfonic acid (TES), 4-(2-hydroxyethyl)-1-piperazine ethanesulfonic acid (HEPES), acetamidoglycine, tricine (N-(2-Hydroxy-1,1-bis(hydroxymethyl)ethyl)glycine), glycinamide, and bicine (2-(Bis(2-hydroxyethyl)amino)acetic acid) buffers. Buffers include other organic acid buffers in addition to the phosphate, citrate, ammonium, acetate, and carbonate buffers explicitly mentioned herein.

An article of manufacture may comprise a container; and a composition contained within the container, wherein the composition comprises a thermostable ligase. An article of manufacture may comprise a container; and a composition contained within the container, wherein the composition comprises a blunt-end ligase. An article of manufacture may comprise a container; and a composition contained within the container, wherein the composition comprises a thermostable blunt-end ligase. The containers may be formed from a variety of materials such as glass or plastic, and may have a sterile access port (for example the container may be an intravenous solution bag or a vial having a stopper pierceable by a hypodermic injection needle). The article of manufacture may further comprise a label or package insert on or associated with the container indicating that the composition may be used to ligate blunt-ended DNA.

Embodiments of methods and compositions provided herein may be described with reference to Figure 1.

The novel thermostable blunt-end DNA ligases disclosed herein are comprised of the fusion of a DNA-binding protein with a thermostable ligase. Such fusion proteins as disclosed herein may further comprise other components, such as, for example, peptide linkers, N-terminal or C-terminal additions, tag peptides, and other amino acid sequences, including D-amino acids and peptide mimetics. In addition, fusion protein as disclosed herein may further comprise other components, such as, for example, sugars (e.g., the fusion proteins may be glycosylated), polymers such as polyethylene glycol (e.g., the fusion proteins may be PEGylated), organic moieties other than amino acids, and other additions linked to the fusion proteins. The DNA-binding protein portion increases the affinity of the ligase to DNA substrates, resulting in enhanced ligation in the challenging conditions of high temperature, blunt-end ligation. Many combinations of binding proteins and ligases are possible. For example, the binding protein may be N-terminal or C-terminal relative to the ligase.

It will be understood that multiple combinations of DNA ligases and DBA-binding proteins may be joined to produce the fusion proteins disclosed herein. For example, the following 16 combinations have been constructed (Table 2):

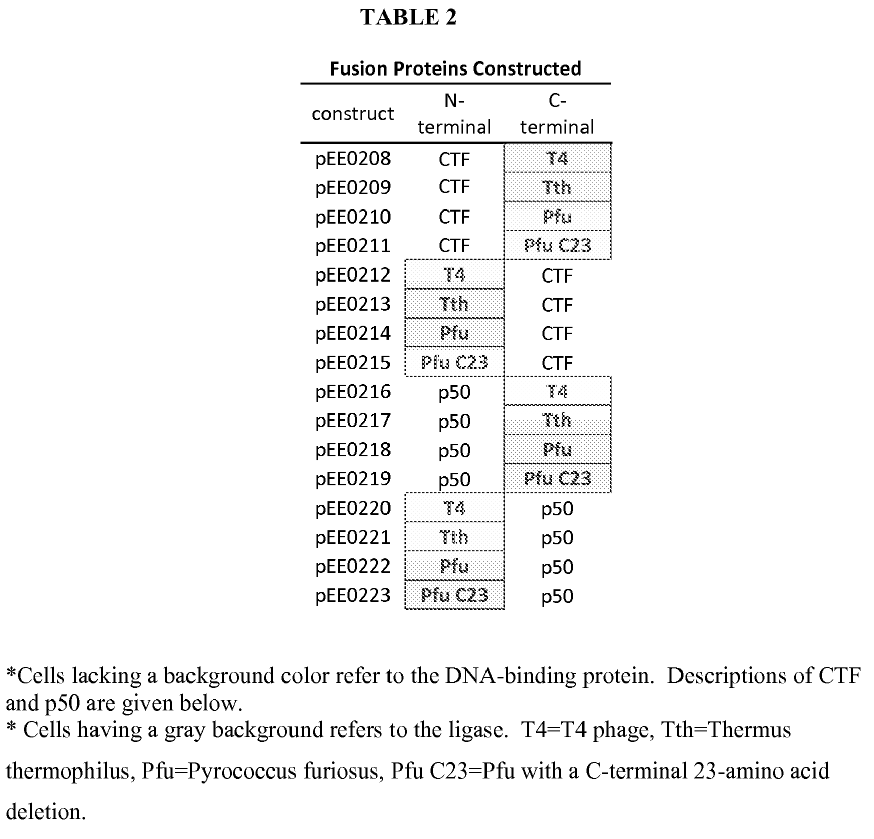

Additional variations are possible, including: (1) different DNA-binding proteins, including thermostable proteins; (2) other DNA ligases, including T4 DNA ligase variants engineered to be thermostable; (3) multiple DNA-binding proteins per ligase, or multiple ligases per DNA-binding protein; (4) transient, non-covalent linkages between the ligase and DNA-binding protein, rather than a protein fusion, to better enable multiple ligation events per ligase molecule; (5) dimers or higher-order multimers of fusion proteins to increase the local concentration of ligase once DNA substrates are bound; (6) different degrees of affinity between the DNA substrate and the DNA-binding protein - for example, using the natural target sequence of p50, GGGAATTCCC (SEQ ID NO: 6), in the DNA target, to enable low picomolar affinity; (7) other nucleic acid modifying enzymes instead of DNA ligase to perform other molecular reactions.

An exemplary method for producing the fusion proteins disclosed herein may be as follows. Fusion proteins may be made by inducing E. coli to express DNA constructs made by standard recombinant DNA methods, followed by standard chromatographic protein purification methods. An affinity tag such as polyhistidine may be employed to assist in the protein purification. In the case of thermostable proteins, purification may be assisted by employing heat to denature most E. coli host proteins. The purified fusion protein may be used in the same way that a standard, non-heat-stable ligase would be used in the application/reaction of choice, for example in a scheme that depends upon ligation of DNA substrates to make a template for amplification.

The methods disclosed herein can be readily incorporated into and used in device for processing a sample, or a system for processing a sample, which may be an automated assay device, or may be an automated assay system. Such assay devices and assay systems may comprise devices and systems disclosed, for example, in U.S. Patent 8,088,593; U.S. Patent 8,380,541; U.S. Pat. Pub. No. US2013/0121530 (App. Ser. No. 13/769,798), filed February 18, 2013; U.S. Pat. Pub. No. US2013/0078485 (App. Ser. No. 13/769,779), filed February 18, 2013; U.S. Pat. Pub. No. US2013/0079236 (App. Ser. No. 13/244,947) filed Sept. 26, 2011; WO 2013/052318 (PCT/US2012/57155), filed September 25, 2012; U.S. Pat. No. 8380541 (Application Serial No. 13/244,946), filed September 26, 2011; U.S. Pat. Pub. No. US2013/0078624 (Patent Application 13/244,949), filed September 26, 2011; and WO 2014/015199 (U.S. Application Serial No. 61/673,245), filed September 26, 2011.

Assay devices and assay systems, including devices for processing a sample and systems for processing a sample, which may be automated assay devices and automated assay systems, may be located and may be used at a point of care location, and may be located and may be used at a plurality of point of care locations. Assay devices and assay systems, including devices for processing a sample and systems for processing a sample, which may be automated assay devices and automated assay systems, may be located and may be used at a point of care location, and may be located and may be used at a plurality of point of service locations.

A sample processing device for performing assays and measurements as disclosed herein may be located, and may perform such assays and measurements, at a point of care location, or a point of service location, or other location. A sample processing system for performing assays and measurements as disclosed herein may be located, and may perform such assays and measurements, at a point of care location, or a point of service location, or other location.

A point of care system may be used at a point of service location, such as a subject's location (e.g., home or business or sports event or security screening or combat location), the location of a healthcare provider (e.g., doctor), a pharmacy or retailer, a clinic, a hospital, an emergency room, a nursing home, a hospice care location, or a laboratory. A retailer may be a pharmacy (e.g., retail pharmacy, clinical pharmacy, hospital pharmacy), drugstore, chain store, supermarket, or grocer. Examples of retailers may include but are not limited to Walgreen's, CVS Pharmacy, Duane Reade, Walmart, Target, Rite Aid, Kroger, Costco, Kaiser Permanente, or Sears. In some situations, a point of service system (including but not limited to point of care system) is deployed at any location that is designated for use by a certifying or licensing entity (e.g., a government certifying entity). In other situations, a point of service system may be used in or embedded in a transportation vehicle, such as a car, boat, truck, bus, airplane, motorcycle, van, traveling medical vehicle, mobile unit, ambulance, fire engine/truck, critical care vehicle, or other vehicle configured to transport a subject from one point to another. A sample collection site may be at a sample acquisition site and/or health assessment and/or treatment locations (which may include any of the sample collection sites described elsewhere herein including but not limited to emergency rooms, doctors' offices, urgent care, tents for screening (which may be in remote locations), a health care professional walking into someone's house to provide home care).

The system (device) or a combination of systems (devices) may be located/positioned at strategic point of service locations. Locations may be selected and optimized based on a variety of objectives, such as but not limited to disease prevalence, rates of disease development, projected disease rates, estimated risk of outbreaks, population demographics, government policies and regulations, customer, physician and patient preferences, access to other technologies at said locations, safety and risk estimates, safety threats, etc. Devices can be relocated on a periodic basis to improve overall utility on a frequent basis, such as daily, weekly, monthly, annually, etc. Systems can be updated to improve performance and/or add functionality. Systems can be updated on a module by module basis. System updates can occur via hardware and/or via software. Systems can be updated with minimal downtime via features enabling blade and/or module extraction and insertion.

Additionally, a point of service location where a sample may be collected from a subject or provided by a subject may be a location remote to an analyzing laboratory. The sample collection site may have a separate facility from the laboratory. The sample may or may not be collected fresh from the subject at the point of service location. Alternatively, the sample may be collected from the subject elsewhere and brought to the point of service location. In some embodiments, no sample preparation step is provided on the sample before being provided to the device. For example, no slide needs to be prepped before a sample is provided to the device. Alternatively, one or more sample preparation step may be performed on the sample before being provided to the device.

A sample collection site at a point of service location may be a blood collection center, or any other bodily fluid collection center. The sample collection site may be a biological sample collection center. In some embodiments, a sample collection site may be a retailer. Other examples of sample collection sites may include hospitals, clinics, health care professionals' offices, schools, day-care centers, health centers, assisted living residences, government offices, traveling medical care units, or the home. For example, a sample collection site may be a subject's home. A sample collection site may be any location where a sample from the subject is received by the device. A collection site may be a moving location, such as on or with a patient or in a mobile unit or vehicle or with a travelling doctor. Any location may be designated as a sample collection site. The designation may be made by any party, including but not limited to the laboratory, entity associated with the laboratory, governmental agency, or regulatory body. Any description herein relating to sample collection site or point of service location may relate to or be applied to retailers, hospitals, clinics, or any other examples provided herein and vice versa.

A device may be part of a system, a component of which may be a sample processing device. A device may be a sample processing device. A sample processing device may be configured to facilitate collection of a sample, prepare a sample for a clinical test, or effect a chemical reaction with one or more reagents or other chemical or physical processing, as disclosed herein. A sample processing device may be configured to obtain data from a sample. A sample processing device may be configured to transmit data obtained from a sample. A sample processing device may be configured to analyze data from a sample. A sample processing device may be configured to communicate with another device, or a laboratory, or an individual affiliated with a laboratory, to analyze data obtained from a sample.

Point of service systems described herein are configured to process samples at a location where the point of service system is accessible by a user. In an example, a point of service system is located at a subject's home and a sample is collected from a subject and processed in the subject's home. In another example, a point of service system is located at a drug store and a sample is collected from a subject and processed in the drug store. In another example, a point of service system is located at the location of a healthcare provider (e.g., doctor's office) and a sample is collected from a subject and processed at the location of the healthcare provider. In another example, a point of service system is located onboard a transportation system (e.g., vehicle) and a sample is collected from a subject and processed on the transportation system.

In some embodiments, post-sample processing analysis, including diagnosis and/or treatment, is performed by the point of service system at the location of the point of service system. In other embodiments, post-sample processing analysis is performed remotely from a location in which a sample is collected and processed. In an example, post-sample processing analysis is performed at the location of a healthcare provider. In another example, post-sample processing analysis is performed at the location of a processing system. In another example, post-sample processing analysis is performed on a server (e.g., on the cloud).

The post-sampling analysis may occur at a laboratory or by an entity affiliated with a laboratory. A laboratory can be an entity or facility capable of performing a clinical test or analyzing collected data. A laboratory can provide controlled conditions in which scientific research, experiments, and measurement can be performed. The laboratory can be a medical laboratory or clinical laboratory where tests can be done on clinical specimens, or analysis can occur on data collected from clinical specimens, in order to get information about the health of a patient as pertaining to the diagnosis, prognosis, treatment, and/or prevention of disease. A clinical specimen may be a sample collected from a subject. Preferably, a clinical specimen may be collected from the subject at a sample collection site that is at a separate facility from the laboratory, as described in further detail elsewhere herein. The clinical specimen may be collected from the subject using a device, which is placed at a designated sample collection site or in or on the subject.

A sample processing device may be configured to be placed in or on a subject. A sample processing device may be configured to accept a sample from a subject, either directly or indirectly. A sample may be, for example, a blood sample (e.g., a sample obtained from a fingerstick, or from venipuncture, or an arterial blood sample), a urine sample, a biopsy sample, a tissue slice, stool sample, or other biological sample; a water sample, a soil sample, a food sample, an air sample; or other sample. A blood sample may comprise, e.g., whole blood, plasma, or serum. A sample processing device may receive a sample from the subject through a housing of the device. The sample collection may occur at a sample collection site, or elsewhere. The sample may be provided to the device at a sample collection site.

In some embodiments, a sample processing device may be configured to accept or hold a cartridge. In some embodiments, a sample processing device may comprise a cartridge. The cartridge may be removable from the sample processing device. In some embodiments, a sample may be provided to the cartridge of the sample processing device. Alternatively, a sample may be provided to another portion of a sample processing device. The cartridge and/or device may comprise a sample collection unit that may be configured to accept a sample.

A cartridge may include a sample, and may include reagents for use in processing or testing a sample, disposables for use in processing or testing a sample, or other materials. Following placement of a cartridge on, or insertion of a cartridge into, a sample processing device, one or more components of the cartridge may be brought into fluid communication with other components of the sample processing device. For example, if a sample is collected at a cartridge, the sample may be transferred to other portions of the sample processing device. Similarly, if one or more reagents are provided on a cartridge, the reagents may be transferred to other portions of the sample processing device, or other components of the sample processing device may be brought to the reagents. In some embodiments, the reagents or components of a cartridge may remain on-board the cartridge. In some embodiments, no fluidics are included that require tubing or that require maintenance (e.g., manual or automated maintenance).

A sample or reagent may be transferred to a device, such as a sample processing device. A sample or reagent may be transferred within a device. Such transfer of sample or reagent may be accomplished without providing a continuous fluid pathway from cartridge to device. Such transfer of sample or reagent may be accomplished without providing a continuous fluid pathway within a device. In embodiments, such transfer of sample or reagent may be accomplished by a sample handling system (e.g., a pipette); for example, a sample, reagent, or aliquot thereof may be aspirated into an open-tipped transfer component, such as a pipette tip, which may be operably connected to a movable unit which transfers the tip, with the sample, reagent, or aliquot thereof contained within the tip, to a location on or within the sample processing device. In some embodiments, a tip from the sample handling system may be inserted at least partially into the sample vessel and/or cavity. The tip may be insertable and removable from the sample vessel and/or cavity. The sample, reagent, or aliquot thereof can be deposited at a location on or within the sample processing device. Sample and reagent, or multiple reagents, may be mixed using a sample handling system in a similar manner. One or more components of the cartridge may be transferred in an automated fashion to other portions of the sample processing device, and vice versa.

A device, such as a sample processing device, may have a fluid handling system (which may be part of a sample handling system). A fluid handling system may perform, or may aid in performing, transport, dilution, extraction, aliquotting, mixing, and other actions with a fluid, such as a sample. In some embodiments, a fluid handling system may be contained within a device housing. A fluid handling system may permit the collection, delivery, processing and/or transport of a fluid, dissolution of dry reagents, mixing of liquid and/or dry reagents with a liquid, as well as collection, delivery, processing and/or transport of non-fluidic components, samples, or materials. The fluid may be a sample, a reagent, diluent, wash, dye, or any other fluid that may be used by the device, and may include, but not limited to, homogenous fluids, different liquids, emulsions, suspensions, and other fluids. A fluid handling system, including without limitation a pipette, may also be used to transport vessels (with or without fluid contained therein) around the device. The fluid handling system may dispense or aspirate a fluid. The sample may include one or more particulate or solid matter floating within a fluid.

In embodiments, a fluid handling system may comprise a pipette, pipette tip, syringe, capillary, or other component. The fluid handling system may have portion with an interior surface and an exterior surface and an open end. The fluid handling system may comprise a pipette, which may include a pipette body and a pipette nozzle, and may comprise a pipette tip. A pipette tip may or may not be removable from a pipette nozzle. In embodiments, a fluid handling system may use a pipette mated with a pipette tip; a pipette tip may be disposable. A tip may form a fluid-tight seal when mated with a pipette. A pipette tip may be used once, twice, or more times. In embodiments, a fluid handling system may use a pipette or similar device, with or without a pipette tip, to aspirate, dispense, mix, transport, or otherwise handle the fluid. The fluid may be dispensed from the fluid handling system when desired. The fluid may be contained within a pipette tip prior to being dispensed, e.g., from an orifice in the pipette tip. In embodiments, or instances during use, all of the fluid may be dispensed; in other embodiments, or instances during use, a portion of the fluid within a tip may be dispensed. A pipette may selectively aspirate a fluid. The pipette may aspirate a selected amount of fluid. The pipette may be capable of actuating stirring mechanisms to mix the fluid within the tip or within a vessel. The pipette may incorporate tips or vessels creating continuous flow loops for mixing, including of materials or reagents that are in non-liquid form. A pipette tip may also facilitate mixture by metered delivery of multiple fluids simultaneously or in sequence, such as in 2-part substrate reactions.

The fluid handling system may include one or more fluidically isolated or hydraulically independent units. For example, the fluid handling system may include one, two, or more pipette tips. The pipette tips may be configured to accept and confine a fluid. The tips may be fluidically isolated from or hydraulically independent of one another. The fluid contained within each tip may be fluidically isolated or hydraulically independent from one fluids in other tips and from other fluids within the device. The fluidically isolated or hydraulically independent units may be movable relative to other portions of the device and/or one another. The fluidically isolated or hydraulically independent units may be individually movable. A fluid handling system may comprise one or more base or support. A base or support may support one or more pipette or pipette units. A base or support may connect one or more pipettes of the fluid handling system to one another.

A sample processing device may be configured to perform processing steps or actions on a sample obtained from a subject. Sample processing may include sample preparation, including, e.g., sample dilution, division of a sample into aliquots, extraction, contact with a reagent, filtration, separation, centrifugation, or other preparatory or processing action or step. A sample processing device may be configured to perform one or more sample preparation action or step on the sample. Optionally, a sample may be prepared for a chemical reaction and/or physical processing step. A sample preparation action or step may include one or more of the following: centrifugation, separation, filtration, dilution, enriching, purification, precipitation, incubation, pipetting, transport, chromatography, cell lysis, cytometry, pulverization, grinding, activation, ultrasonication, micro column processing, processing with magnetic beads, processing with nanoparticles, or other sample preparation action or steps. For example, sample preparation may include one or more step to separate blood into serum and/or particulate fractions, or to separate any other sample into various components. Sample preparation may include one or more step to dilute and/or concentrate a sample, such as a blood sample, or other biological samples. Sample preparation may include adding an anti-coagulant or other ingredients to a sample. Sample preparation may also include purification of a sample. In embodiments, all sample processing, preparation, or assay actions or steps are performed by a single device. In embodiments, all sample processing, preparation, or assay actions or steps are performed within a housing of a single device. In embodiments, most sample processing, preparation, or assay actions or steps are performed by a single device, and may be performed within a housing of a single device. In embodiments, many sample processing, preparation, or assay actions or steps are performed by a single device, and may be performed within a housing of a single device. In embodiments, sample processing, preparation, or assay actions or steps may be performed by more than one device.

A sample processing device may be configured to run one or more assay on a sample, and to obtain data from the sample. An assay may include one or more physical or chemical treatments, and may include running one or more chemical or physical reactions. A sample processing device may be configured to perform one, two or more assays on a small sample of bodily fluid. One or more chemical reaction may take place on a sample having a volume, as described elsewhere herein. For example one or more chemical reaction may take place in a pill having less than femtoliter volumes. In an instance, the sample collection unit is configured to receive a volume of the bodily fluid sample equivalent to a single drop or less of blood or interstitial fluid. In embodiments, the volume of a sample may be a small volume, where a small volume may be a volume that is less than about 1000 µL, or less than about 500 µL, or less than about 250 µL, or less than about 150 µL, or less than about 100 µL, or less than about 75 µL, or less than about 50 µL, or less than about 40 µL, or less than about 20 µL, or less than about 10 µL, or other small volume. In embodiments, all sample assay actions or steps are performed on a single sample. In embodiments, all sample assay actions or steps are performed by a single device. In embodiments, all sample assay actions or steps are performed within a housing of a single device. In embodiments, most sample assay actions or steps are performed by a single device, and may be performed within a housing of a single device. In embodiments, many sample assay actions or steps are performed by a single device, and may be performed within a housing of a single device. In embodiments, sample processing, preparation, or assay actions or steps may be performed by more than one device.

A sample processing device may be configured to perform a plurality of assays on a sample. In embodiments, a sample processing device may be configured to perform a plurality of assays on a single sample. In embodiments, a sample processing device may be configured to perform a plurality of assays on a single sample, where the sample is a small sample. For example, a small sample may have a sample volume that is a small volume of less than about 1000 µL, or less than about 500 µL, or less than about 250 µL, or less than about 150 µL, or less than about 100 µL, or less than about 75 µL, or less than about 50 µL, or less than about 40 µL, or less than about 20 µL, or less than about 10 µL, or other small volume. A sample processing device may be capable of performing multiplexed assays on a single sample. A plurality of assays may be run simultaneously; may be run sequentially; or some assays may be run simultaneously while others are run sequentially. One or more control assays and/or calibrators (e.g., including a configuration with a control of a calibrator for the assay/tests) can also be incorporated into the device; control assays and assay on calibrators may be performed simultaneously with assays performed on a sample, or may be performed before or after assays performed on a sample, or any combination thereof. In embodiments, all sample assay actions or steps are performed by a single device. In embodiments, all of a plurality of assay actions or steps are performed within a housing of a single device. In embodiments, most sample assay actions or steps, of a plurality of assays, are performed by a single device, and may be performed within a housing of a single device. In embodiments, many sample assay actions or steps, of a plurality of assays, are performed by a single device, and may be performed within a housing of a single device. In embodiments, sample processing, preparation, or assay actions or steps may be performed by more than one device.

In embodiments, all of a plurality of assays may be performed in a short time period. In embodiments, such a short time period comprises less than about three hours, or less than about two hours, or less than about one hour, or less than about 40 minutes, or less than about 30 minutes, or less than about 25 minutes, or less than about 20 minutes, or less than about 15 minutes, or less than about 10 minutes, or less than about 5 minutes, or less than about 4 minutes, or less than about 3 minutes, or less than about 2 minutes, or less than about 1 minute, or other short time period.

A device may be capable of performing all on-board steps (e.g., steps or actions performed by a single device) in a short amount of time. A device may be capable of performing all on-board steps on a single sample in a short amount of time. For example, from sample collection from a subject to transmitting data and/or to analysis may take about 3 hours or less, 2 hours or less, 1 hour or less, 50 minutes or less, 45 minutes or less, 40 minutes or less, 30 minutes or less, 20 minutes or less, 15 minutes or less, 10 minutes or less, 5 minutes or less, 4 minutes or less, 3 minutes or less, 2 minutes or less, or 1 minute or less. The amount of time from accepting a sample within the device to transmitting data and/or to analysis from the device regarding such a sample may depend on the type or number of steps, tests, or assays performed on the sample. The amount of time from accepting a sample within the device to transmitting data and/or to analysis from the device regarding such a sample may take about 3 hours or less, 2 hours or less, 1 hour or less, 50 minutes or less, 45 minutes or less, 40 minutes or less, 30 minutes or less, 20 minutes or less, 15 minutes or less, 10 minutes or less, 5 minutes or less, 4 minutes or less, 3 minutes or less, 2 minutes or less, or 1 minute or less.

A device or system as disclosed herein, such as, e.g., a point of service device or a point of service system, may process a sample according to a test order or schedule. In some situations, a feedback loop (coupled with sensors) enables the point of service device or system to monitor the progress of sample processing and maintain or alter the test order or schedule. In an example, if the device or system detects that processing is taking longer than the predetermined amount of time set forth in the schedule, the device or system speeds up processing or adjusts any parallel processes, such as sample processing in another module of the device or system. The feedback loop permits real-time or pseudo-real time (e.g., cached) monitoring. In some situations, the feedback loop may provide permit reflex testing, which may cause subsequent tests, assays, preparation steps, and/or other processes to be initiated after starting or completing another test and/or assay or sensing one or more parameter. Such subsequent tests, assays, preparation steps, and/or other processes may be initiated automatically without any human intervention.

In some embodiments, the point of service system may stick to a predetermined test order or schedule based on initial parameters and/or desired effects. In other embodiments, the schedule and/or test order may be modified on the fly. The schedule and/or test order may be modified based on one or more detected conditions, one or more additional processes to run, one or more processes to no longer run, one or more processes to modify, one or more resource/component utilization modifications, one or more detected error or alert condition, one or more unavailability of a resource and/or component, one or more subsequent input or sample provided by a user, external data, or any other reason.

In some examples, one or more additional samples may be provided to a device after one or more initial samples are provided to the device. The additional samples may be from the same subject or different subjects. The additional samples may be the same type of sample as the initial sample or different types of samples (e.g., blood, tissue). The additional samples may be provided prior to, concurrently with, and/or subsequent to processing the one or more initial samples on the device. The same and/or different tests or desired criteria may be provided for the additional samples, as opposed to one another and/or the initial samples. The additional samples may be processed in sequence and/or in parallel with the initial samples. The additional samples may use one or more of the same components as the initial samples, or may use different components. The additional samples may or may not be requested in view of one or more detected condition of the initial samples.

In some embodiments, the system accepts a sample with the aid of a sample collection module, such as a lancet, scalpel, or fluid collection vessel. The system then loads or accesses a protocol for performing one or more processing routines from a plurality of potential processing routines. In an example, the system loads a centrifugation protocol and cytometry protocol. In some embodiments, the protocol may be loaded from an external device to a sample processing device. Alternatively, the protocol may already be on the sample processing device. The protocol may be generated based on one or more desired criteria and/or processing routines. In one example, generating a protocol may include generating a list of one or more subtasks for each of the input processes. In some embodiments, each subtask is to be performed by a single component of the one or more devices. Generating a protocol may also include generating the order of the list, the timing and/or allocating one or more resources.

In an embodiment, a protocol provides processing details or specifications that are specific to a sample or a component in the sample. For instance, a centrifugation protocol may include rotational velocity and processing time that is suited to a predetermined sample density, which enables density-dependent separation of a sample from other material that may be present with a desirable component of the sample.

A protocol is included in the system, such as in a protocol repository of the system, or retrieved from another system, such as a database, in communication with the system. In an embodiment, the system is in one-way communication with a database server that provides protocols to the system upon request from the system for one or more processing protocols. In another embodiment, the system is in two-way communication with a database server, which enables the system to upload user-specific processing routines to the database server for future use by the user or other users that may have use for the user-specific processing routines.

In some cases, a processing protocol is adjustable by a user. In an embodiment, a user may generate a processing protocol with the aid of a protocol engine that provides the user one or more options geared toward tailoring the protocol for a particular use. The tailoring may occur prior to use of the protocol. In some embodiments, the protocol may be modified or updated while the protocol is in use.

With the aid of a protocol, a system processes a sample, which may include preparing the sample, assaying the sample and detecting one or more components of interest in the sample. In some cases, the system performs data analysis with respect to the sample or a plurality of sample after processing. In other cases, the system performs data analysis during processing. In some embodiments, data analysis is performed on-board--that is, on the system. In other embodiments, data analysis is performed using a data analysis system that is external to the system. In such a case, data is directed to the analysis system while the sample is being processed or following processing.

A device may be configured to prepare a sample for disposal, or to dispose of a sample, such as a biological sample, following processing or assaying of a sample.

In embodiments, a sample processing device may be configured to transmit data obtained from a sample. In embodiments, a sample processing device may be configured to communicate over a network. A sample processing device may include a communication module that may interface with the network. A sample processing device may be connected to the network via a wired connection or wirelessly. The network may be a local area network (LAN) or a wide area network (WAN) such as the Internet. In some embodiments, the network may be a personal area network. The network may include the cloud. The sample processing device may be connected to the network without requiring an intermediary device, or an intermediary device may be required to connect a sample processing device to a network. A sample processing device may communicate over a network with another device, which may be any type of networked device, including but not limited to a personal computer, server computer, or laptop computer; personal digital assistants (PDAs) such as a Windows CE device; phones such as cellular phones, smartphones (e.g., iPhone, Android, Blackberry, etc.), or location-aware portable phones (such as GPS); a roaming device, such as a network-connected roaming device; a wireless device such as a wireless email device or other device capable of communicating wireless with a computer network; or any other type of network device that may communicate possibly over a network and handle electronic transactions. Such communication may include providing data to a cloud computing infrastructure or any other type of data storage infrastructure which may be accessed by other devices.

A sample processing device may provide data regarding a sample to, e.g., a health care professional, a health care professional location, such as a laboratory, or an affiliate thereof. One or more of a laboratory, health care professional, or subject may have a network device able to receive or access data provided by the sample processing device. A sample processing device may be configured to provide data regarding a sample to a database. A sample processing device may be configured to provide data regarding a sample to an electronic medical records system, to a laboratory information system, to a laboratory automation system, or other system or software. A sample processing device may provide data in the form of a report.

A laboratory, device, or other entity or software may perform analysis on data regarding a sample in real-time. A software system may perform chemical analysis and/or pathological analysis, or these could be distributed amongst combinations of lab, clinical, and specialty or expert personnel. Analysis may include qualitative and/or quantitative evaluation of a sample. Data analysis may include a subsequent qualitative and/or quantitative evaluation of a sample. Optionally, a report may be generated based on raw data, preprocessed data, or analyzed data. Such a report may be prepared so as to maintain confidentiality of the data obtained from the sample, the identity and other information regarding the subject from whom a sample was obtained, analysis of the data, and other confidential information. The report and/or the data may be transmitted to a health care professional. Data obtained by a sample processing device, or analysis of such data, or reports, may be provided to a database, an electronic medical records system, to a laboratory information system, to a laboratory automation system, or other system or software.

### EXAMPLES

### EXAMPLE 1

Examples of individual protein components suitable for use in providing fusion proteins as disclosed herein include (the amino acid sequences are provided using the one-letter code for amino acids):

**TABLE 3**

| Protein Name | Protein Sequence | SEQ ID NO: |
|---|---|---|
| **p50 DNA-binding protein** (fragment from the human NF-kappa-B protein, accession number NP_003989, amino acids 40-366) | adgpylqileqpkqrgfrfryvcegpshgglpgasseknkksy pqvkicnyvgpakvivqlvtngknihlhahslvgkhcedgict vtagpkdmvvgfanlgilhvtkkkvfetlearmteacirgynp gllvhpdlaylqaegggdrqlgdrekelirqaalqqtkemdls vvrlmftaflpdstgsftrrlepvvsdaiydskapnasnlkiv rmdrtagcvtggeeiyllcdkvqkddiqirfyeeeenggvweg fgdfsptdvhrqfaivfktpkykdinitkpasvfvqlrrksdl etsepkpflyypeikdkeevqrkrqk | 2 |
| **Tth DNA ligase** (Thermus Thermophilus strain HB8, accession YP_144363.1) | mtleearkrvnelrdliryhnyryyvladpeisdaeydrllre lkeleerfpelkspdsptlqvgarpleatfrpvrhptrmysld nafnldelkafeerieralgrkgpfaytvehkvdglsvnlyye egvlvygatrgdgevgeevtqnlltiptiprrlkgvperlevr gevympieaflrlneeleergerifknprnaaagslrqkdpri takrglratfyalglgleeveregvatqfallhwlkekgfpve hgyaravgaegveavyqdwlkkrralpfeadgvvvkldelalw relgytaraprfaiaykfpaeeketrlldvvfqvgrtgrvtpv gilepvflegsevsrvtlhnesyieeldirigdwvlvhkaggv ipevlrvlkerrtgeerpirwpetcpecghrllkegkvhrcpn | 3 |
| | plcpakrfeairhfasrkamdiqglgeklierllekglvkdva dlyrlrkedlvglermgeksaqnllrqieeskkrglerllyal glpgvgevlarnlaarfgnmdrlleasleelleveevgeltar aailetlkdpafrdlvrrlkeagvemeakekggealkgltfvit gelsrpreevkallrrlgakvtdsvsrktsylvvgenpgskle karalgvptlteeelyrlleartgkkaeelv | |
| His10-containing leader | mghhhhhhhhhhssghiegras | 4 |
| Flexible glycine-rich sequence | gssgtsgggsggg | 5 |
| **CTF DNA-binding protein** (a hybrid from the murine NFATc1 protein, accession number NP_058071, amino acids 390-506; followed by an alanine spacer; followed by a fragment from the human NF-kappa-B protein, accession number NP_003989, amino acids 249-366) | spptsymspslpaldwqlpshsgpyelrievqpkshhrahyete gsrgavkasagghpivqlhgylenepltlqlfigtaddrllrp hafyqvhritgktvsttsheiilsntkvleipllpennmraii dcagilklrnsdielrkgetdigrkntrvrlvfrvhipqpngr tlslqasnlkivrmdrtagcvtggeeiyllcdkvqkddiqirf yeeeenggvwegfgdfsptdvhrqfaivfktpkykdinitkpa svfvqlrrksdletsepkpflyypeikdkeevqrkrqk | 7 |
| **T4 DNA ligase** (accession number NP_049813) | milkilneiasigstkqkqaileknkdnellkrvyrltysrgl qyyikkwpkpgiatqsfgmltltdmldfieftlatrkltgnaa ieeltgyitdgkkddvevlrrvmmrdlecgasvsiankvwpgl ipeqpqmlassydekginknikfpafaqlkadgarcfaevrgd elddvrllsragneylgldllkeelikmtaearqihpegvlid gelvyheqvkkepegldflfdaypenskakefaevaesrtasn gianksl kgtisekeaqcmkfqvwdyvplveiyslpafrlkyd vrfskleqmtsgydkvilienqvvnnldeakviykkyidqgle giilknidglwenarsknlykfkevidvdlkivgiyphrkdpt kaggfilesecgkikvnagsglkdkagvksheldrtrimenqn yyigkilececngwlksdgrtdyvklflpiairlredktkant fedvfgdfhevtgl | 8 |
| **Pfu DNA ligase** (Pyrococcus furiosus strain ST04, accession YP_006355162) | mrylelaqlyqklekttmkliktrlvadflkkvpddhlefipy lilgdvfpewderelgvgekllikavamatgidaneiensvkd tgdlgesialavkkrkqksffsqpltikrvyqtlvkvaettge gsqekkmkylanlfmdaepieakyiartvlgtmrtgvaegllr daialafhvkvelveraymltsdfgfvakvaklegneglakvq vqigkpikpmlaqqaanikeallemggeaefeikydgarvqvh kdgdkiivysrrlenvtraipeivealkqsvkpnkaivegelv aigedgrplpfqyvlrrfrrkhniqemmkkiplelnlfdvlyv dgesmidvkfidrrkkleeiiepngkikvaenlitkkveeaea fykkalemgheglmakrldatyepgnrgkkwlkikptmenldl viigaewgegrrahllgsfilgaydpetgeflevgkvgsgftd edlveftkmlkpliikeegkrvwiepkivievtyqeiqkspky ksgfalrfpryvalrddkgpedadtieriaqlyelqermkgkv | 9 |
| **Pfu C23 DNA ligase** (C-terminal 23-amino acid deletion of Pfu) | mrylelaqlyqklekttmkliktrlvadflkkvpddhlefipy lilgdvfpewderelgvgekllikavamatgidaneiensvkd tgdlgesialavkkrkqksffsqpltikrvyqtlvkvaettge gsqekkmkylanlfmdaepieakyiartvlgtmrtgvaegllr daialafhvkvelveraymltsdfgfvakvaklegneglakvq vqigkpikpmlaqqaanikeallemggeaefeikydgarvqvh kdgdkiivysrrlenvtraipeivealkqsvkpnkaivegelv aigedgrplpfqyvlrrfrrkhniqemmkkiplelnlfdvlyv dgesmidvkfidrrkkleeiiepngkikvaenlitkkveeaea fykkalemgheglmakrldatyepgnrgkkwlkikptmenldl viigaewgegrrahllgsfilgaydpetgeflevgkvgsgftd | 10 |
| | edlveftkmlkpliikeegkrvwiepkiviievtyqeiqkspky ksgfalrfpryvalrddkgp | |
| HIS-rich leader, p50 DNA-binding protein, glycine-rich linker | Mghhhhhhhhhhssghiegrasadgpylqileqpkqrgfrfry vcegpshgglpgasseknkksypqvkicnyvgpakvivqlvtn gknihlhahslvgkhcedgictvtagpkdmvvgfanlgilhvt kkkvfetlearmteacirgynpgllvhpdlaylqaegggdrql gdrekelirqaalqqtkemdlsvvrlmftaflpdstgsftrrl epvvsdaiydskapnasnlkivrmdrtagcvtggeeiyllcdk vqkddiqirfyeeeenggvwegfgdfsptdvhrqfaivfktpk ykdinitkpasvfvqlrrksdletsepkpflyypeikdkeevq rkrqkgssgtsgggsggg | 11 |
| HIS-rich leader, CTF DNA-binding protein, glycine-rich linker | Mghhhhhhhhhhssghiegrassptsymspslpaldwqlpshs gpyelrievqpkshhrahyetegsrgavkasagghpivqlhgy leneplltlqlfigtaddrllrphafyqvhritgktvsttshei ilsntkvleipllpennmraiidcagilklrnsdielrkgetd igrkntrvrlvfrvhipqpngrtlslqasnlkivrmdrtagcv tggeeiyllcdkvqkddiqirfyeeeenggvwegfgdfsptdv hrqfaivfktpkykdinitkpasvfvqlrrksdletsepkpfl yypeikdkeevqrkrqkgssgtsgggsggg | 12 |

The following provides an example of a fusion protein providing thermostable blunt-end DNA ligase activity:
**p50-Tth ligase fusion,** Theranos construct EE0217, e.g. protein prep RDP0078, consists of a His10-containing leader (mghhhhhhhhhhssghiegras, SEQ ID NO: 4), *p50* (shown below in *italic* text, beginning with *adgpy...* and ending with ... *rkrqk,* SEQ ID NO: 2), a flexible glycine-rich sequence (gssgtsgggsggg, SEQ ID NO: 5), and the Tth DNA ligase (shown below in underlined text, SEQ ID NO: 3). This fusion protein has the following sequence (SEQ ID NO: 1):

mghhhhhhhhhhssghiegrasadgpylqileqpkqrgfrfryvcegpshgglpgasseknkksypqvkicnyvgpakvivql
vtngknihlhahslvgkhcedgictvtagpkdmvvgfanlgilhvtkkkvfetlearmteacirgynpgllvhpdlaylqaegggd
rqlgdrekelirqaalqqtkemdlsvvrlmftaflpdstgsftrrlepvvsdaiydskapnasnlkivrmdrtagcvtggeeiyllcdk
vqkddiqirfyeeeenggvwegfgdfsptdvhrqfaivfktpkykdinitkpasvfvqlrrksdletsepkpflyypeikdkeevqrk
rqkgssgtsgggsgggmtleearkrvnelrdliryhnyryyvladpeisdaeydrllrelkeleerfpelkspdsptlqvgarpleatfr
pvrhptrmysldnafnldelkafeerieralgrkgpfaytvehkvdglsvnlyyeegvlvygatrgdgevgeevtqnlltiptiprrlk
gvperlevrgevympieaflrlneeleergerifknprnaaagslrqkdpritakrglratfyalglgleeveregvatqfallhwlkek
gfpvehgyaravgaegveavyqdwlkkrralpfeadgvvvkldelalwrelgytaraprfaiaykfpaeeketrlldvvfqvgrtgr
vtpvgilepvflegsevsrvtlhnesyieeldirigdwvlvhkaggvipevlrvlkerrtgeerpirwpetcpecghrllkegkvhrcp
nplcpakrfeairhfasrkamdiqglgeklierllekglvkdvadlyrlrkedlvglermgeksaqnllrqieeskkrglerllyalglpg
vgevlarnlaarfgnmdrlleasleelleveevgeltarailetlkdpafrdlvrrlkeagvemeakekggealkgltfvitgelsrpre
evkallrrlgakvtdsvsrktsylvvgenpgsklekaralgvptlteeelyrlleartgkkaeelv

The fusion protein sequence SEQ ID NO: 1 presented above is an example of a fusion protein that may be represented as "4-2-5-3" in which "4" represents SEQ ID NO: 4; "2" represents SEQ ID NO: 2; "5" represents SEQ ID NO: 5; and "3" represents SEQ ID NO: 3. Such representations indicate, in a N-terminal to C-terminal orientation, linear fusion proteins comprised of fusions of the amino acid sequence indicated by the numbers (which themselves indicate the corresponding SEQ ID NO:s). Further such representations of fusion proteins are presented below.

An example of a blunt-ended ligation reaction performed at 75°C is provided below. The DNA substrate was a 49-bp duplex DNA made by annealing oligonucleotides EE0139 (SEQ ID NO: 20) and EE0140 (SEQ ID NO: 21). This blunt-ended duplex was capable of making concatamers upon multiple ligation events. The results of the reaction products were separated by size on an agarose gel, as shown in Fig. 1, demonstrate that: (1) Tth ligase alone (lanes 2-3), although it is known to be capable of sealing DNA nicks at 75°C, performed very little or no blunt ligation in these conditions; (2) T4 DNA ligase with an N-terminal p50 fusion (lanes 4-5) also performed very little blunt ligation in these conditions, although there was some evidence of more ligation than for Tth alone. This observation was believed to be surprising given the temperature sensitivity of T4 ligase alone; (3) Tth DNA ligase with an N-terminal p50 fusion (lanes 6-7) demonstrated a much higher level of blunt-end ligation at 75°C. Thus, the results shown in Figure 1 demonstrate that fusion proteins as disclosed herein provide improved blunt-end ligation activity at high temperature. Fusion proteins homologous to SEQ ID NO: 1 are believed to provide improved blunt-end ligation activity at high temperature. Thus, fusion proteins having greater than 90 %, or greater than 95% or greater than 99%, sequence homology to SEQ ID NO: 1 are believed to provide blunt-end DNA ligation activity at high temperature.

### EXAMPLE 2

The fusion protein of SEQ ID NO: 1 (and homologous molecules) is an example of a thermostable blunt-end DNA ligase. Examples of further fusion proteins having similar structural characteristics are listed in the following table (Table 4). It is believed that these further fusion proteins share not only structural characteristics, but also functional characteristics as well; thus, the fusion proteins listed in Table 4 are believed to be thermostable blunt-end ligases. In addition, proteins homologous to the fusion proteins of Table 4 believed to be thermostable blunt-end ligases. The first-listed example, SEQ ID NO: 1, has been discussed above, and results of ligation experiments using SEQ ID NO: 1 are shown in Fig. 1. As discussed above, SEQ ID NO: 1 is a combination of a HIS10-containing leader sequence (SEQ ID NO: 4), the DNA-binding protein p50 (SEQ ID NO: 2), a flexible glycine-rich sequence (SEQ ID NO: 5) and Tth DNA ligase (SEQ ID NO: 3), in which the amino acid sequence SEQ ID NO: 4 is covalently linked to the amino acid sequence SEQ ID NO: 2 which is covalently linked to the amino acid sequence SEQ ID NO: 5 which is covalently linked to the amino acid sequence SEQ ID NO: 3; this combination is termed "4-2-5-3" in the left-most column of Table 4. (The term "4-2-5-3" indicates a linear fusion protein in which sequences SEQ ID NO: 4, SEQ ID NO: 2, SEQ ID NO: 5, and SEQ ID NO: 3 are linked as written, from N-terminal (left) to C-terminal (right).) Further combinations of such components illustrate some of the many possible fusion proteins having features as disclosed herein. The other fusion proteins listed in Table 4 include further combinations of leader sequences (SEQ ID NO: 4) with a DNA-binding protein (e.g., SEQ ID NO: 2 or SEQ ID NO: 7), a flexible glycine-rich sequence (SEQ ID NO: 5), and a DNA ligase (SEQ ID NO: 3, SEQ ID NO: 8, SEQ ID NO: 9, or SEQ ID NO: 10). Thus, exemplary fusion proteins disclosed in Table 4 include an amino acid sequence of a histidine-rich leader sequence, a DNA ligase, and a flexible glycine-rich linker sequence (i.e., SEQ ID NO: 11 or SEQ ID NO: 12) covalently linked to a ligase (e.g., SEQ ID NO: 3, SEQ ID NO: 8, SEQ ID NO: 9, or SEQ ID NO: 10). These exemplary fusion proteins are believed to provide yet further thermostable blunt-ended nucleic acid ligases.

**TABLE 4**

| Fusion Protein Formed by SEQ ID NOs: | Protein Sequence | SEQ ID NO: |
|---|---|---|
| 4-2-5-3 | mghhhhhhhhhhssghiegrasadgpylqileqpkqrgfrfryvcegps hgglpgasseknkksypqvkicnyvgpakvivqlvtngknihlhahslv gkhcedgictvtagpkdmvvgfanlgilhvtkkkvfetlearmteacir gynpgllvhpdlaylqaegggdrqlgdrekelirqaalqqtkemdlsvv rlmftaflpdstgsftrrlepvvsdaiydskapnasnlkivrmdrtagc vtggeeiyllcdkvqkddiqirfyeeeenggvwegfgdfsptdvhrqfa ivfktpkykdinitkpasvfvqlrrksdletsepkpflyypeikdkeev qrkrqkgssgtsgggsgggmtleearkrvnelrdliryhnyryyvladp eisdaeydrllrelkeleerfpelkspdsptlqvgarpleatfrpvrhp trmysldnafnldelkafeerieralgrkgpfaytvehkvdglsvnlyy eegvlvygatrgdgevgeevtqnlltiptiprrlkgvperlevrgevym pieaflrlneeleergerifknprnaaagslrqkdpritakrglratfy alglgleeveregvatqfallhwlkekgfpvehgyaravgaegveavyq dwlkkrralpfeadgvvvkldelalwrelgytaraprfaiaykfpaeek etrlldvvfqvgrtgrvtpvgilepvflegsevsrvtlhnesyieeldi rigdwvlvhkaggvipevlrvlkerrtgeerpirwpetcpecghrllke gkvhrcpnplcpakrfeairhfasrkamdiqglgeklierllekglvkd vadlyrlrkedlvglermgeksaqnllrqieeskkrglerllyalglpg vgevlarnlaarfgnmdrlleasleelleveevgeltarailetlkdpa frdlvrrlkeagvemeakekggealkgltfvitgelsrpreevkallrr lgakvtdsvsrktsylvvgenpgsklekaralgvptlteelyrllear tgkkaeelv | 1 |
| 4-2-5-8 | Mghhhhhhhhhssghiegrasadgpylqileqpkqrgfrfryvcegps hgglpgasseknkksypqvkicnyvgpakvivqlvtngknihlhahslv gkhcedgictvtagpkdmvvgfanlgilhvtkkkvfetlearmteacir gynpgllvhpdlaylqaegggdrqlgdrekelirqaalqqtkemdlsvv rlmftaflpdstgsftrrlepvvsdaiydskapnasnlkivrmdrtagc vtggeeiyllcdkvqkddiqirfyeeeenggvwegfgdfsptdvhrqfa ivfktpkykdinitkpasvfvqlrrksdletsepkpflyypeikdkeev qrkrqkgssgtsgggsgggmilkilneiasigstkqkqaileknkdnel | 13 |
| | lkrvyrltysrglqyyikkwpkpgiatqsfgmltltdmldfieftlatr kltgnaaieeltgyitdgkkddvevlrrvmmrdlecgasvsiankvwpg lipeqpqmlassydekginknikfpafaqlkadgarcfaevrgdelddv rllsragneylgldllkeelikmtaearqihpegvlidgelvyheqvkk epegldflfdaypenskakefaevaesrtasngianksl kgtisekeaq cmkfqvwdyvplveiyslpafrlkydvrfskleqmtsgydkvilienqv vnnldeakviykkyidqglegiilknidglwenarsknlykfkevidvd lkivgiyphrkdptkaggfilesecgkikvnagsglkdkagvksheldr trimenqnyyigkilecec ngwlksdgrtdyvklflpiairlredktka ntfedvfgdfhevtgl | |
| 4-2-5-9 | Mghhhhhhhhhhssghiegrasadgpylqileqpkqrgfrfryvcegps hgglpgasseknkksypqvkicnyvgpakvivqlvtngknihlhahslv gkhcedgictvtagpkdmvvgfanlgilhvtkkkvfetlearmteacir gynpgllvhpdlaylqaegggdrqlgdrekelirqaalqqtkemdlsvv rlmftaflpdstgsftrrlepvvsdaiydskapnasnlkivrmdrtagc vtggeeiyllcdkvqkddiqirfyeeeenggvwegfgdfsptdvhrqfa ivfktpkykdinitkpasvfvqlrrksdletsepkpflyypeikdkeev qrkrqkgssgtsgggsgggmrylelaqlyqklekttmkliktrlvadfl kkvpddhlefipylilgdvfpewderelgvgekllikavamatgidane iensvkdtgdlgesialavkkrkqksffsqpltikrvyqtlvkvaettg egsqekkmkylanlfmdaepieakyiartvlgtmrtgvaegllrdaial afhvkvelveraymltsdfgfvakvaklegneglakvqvqigkpikpml aqqaanikeallemggeaefeikydgarvqvhkdgdkiivysrrlenvt raipeivealkqsvkpnkaivegelvaigedgrplpfqyvlrrfrrkhn iqemmkkiplelnlfdvlyvdgesmidvkfidrrkkleeiiepngkikv aenlitkkveeaeafykkalemgheglmakrldatyepgnrgkkwlkik ptmenldlviigaewgegrrahllgsfilgaydpetgeflevgkvgsgf tdedlveftkmlkpliikeegkrvwiepkivievtyqeiqkspkyksgf alrfpryvalrddkgpedadtieriaqlyelqermkgkv | 14 |
| 4-2-5-10 | mghhhhhhhhhhssghiegrasadgpylqileqpkqrgfrfryvcegps hgglpgasseknkksypqvkicnyvgpakvivqlvtngknihlhahslv gkhcedgictvtagpkdmvvgfanlgilhvtkkkvfetlearmteacir gynpgllvhpdlaylqaegggdrqlgdrekelirqaalqqtkemdlsvv rlmftaflpdstgsftrrlepvvsdaiydskapnasnlkivrmdrtagc vtggeeiyllcdkvqkddiqirfyeeeenggvwegfgdfsptdvhrqfa ivfktpkykdinitkpasvfvqlrrksdletsepkpflyypeikdkeev qrkrqkgssgtsgggsgggmrylelaqlyqklekttmkliktrlvadfl kkvpddhlefipylilgdvfpewderelgvgekllikavamatgidane iensvkdtgdlgesialavkkrkqksffsqpltikrvyqtlvkvaettg egsqekkmkylanlfmdaepieakyiartvlgtmrtgvaegllrdaial afhvkvelveraymltsdfgfvakvaklegneglakvqvqigkpikpml aqqaanikeallemggeaefeikydgarvqvhkdgdkiivysrrlenvt raipeivealkqsvkpnkaivegelvaigedgrplpfqyvlrrfrrkhn iqemmkkiplelnlfdvlyvdgesmidvkfidrrkkleeiiepngkikv aenlitkkveeaeafykkalemgheglmakrldatyepgnrgkkwlkik ptmenldlviigaewgegrrahllgsfilgaydpetgeflevgkvgsgf tdedlveftkmlkpliikeegkrvwiepkivievtyqeiqkspkyksgf alrfpryvalrddkgp | 15 |
| 4-7-5-3 | Mghhhhhhhhhhssghiegrasssptsymspslpaldwqlpshsgpyelr ievqpkshhrahyetegsrgavkasagghpivqlhgylenepltlqlfi gtaddrllrphafyqvhritgktvsttsheiilsntkvleipllpennm raiidcagilklrnsdielrkgetdigrkntrvrlvfrvhipqpngrtl slqasnlkivrmdrtagcvtggeeiyllcdkvqkddiqirfyeeeengg vwegfgdfsptdvhrqfaivfktpkykdinitkpasvfvqlrrksdlet sepkpflyypeikdkeevqrkrqkgssgtsgggsgggmtleearkrvne lrdliryhnyryyvladpeisdaeydrllrelkeleerfpelkspdspt lqvgarpleatfrpvrhptrmysldnafnldelkafeerieralgrkgp faytvehkvdglsvnlyyeegvlvygatrgdgevgeevtqnlltiptip rrlkgvperlevrgevympieaflrlneeleergerifknprnaaagsl rqkdpritakrglratfyalglgleeveregvatqfallhwlkekgfpv ehgyaravgaegveavyqdwlkkrralpfeadgvvvkldelalwrelgy | 16 |
| | taraprfaiaykfpaeeketrlldvvfqvgrtgrvtpvgilepvflegs evsrvtlhnesyieeldirigdwvlvhkaggvipevlrvlkerrtgeer pirwpetcpecghrllkegkvhrcpnplcpakrfeairhfasrkamdiq glgeklierllekglvkdvadlyrlrkedlvglermgeksaqnllrqie eskkrglerllyalglpgvgevlarnlaarfgnmdrlleasleelleve evgeltarailetlkdpafrdlvrrlkeagvemeakekggealkgltfv itgelsrpreevkallrrlgakvtdsvsrktsylvvgenpgsklekara lgvptlteeelyrlleartgkkaeelv | |
| 4-7-5-8 | Mghhhhhhhhhhssghiegrasssptsymspslpaldwqlpshsgpyelr ievqpkshhrahyetegsrgavkasagghpivqlhgylenepltlqlfi gtaddrllrphafyqvhritgktvsttsheiilsntkvleipllpennm raiidcagilklrnsdielrkgetdigrkntrvrlvfrvhipqpngrtl slqasnlkivrmdrtagcvtggeeiyllcdkvqkddiqirfyeeeengg vwegfgdfsptdvhrqfaivfktpkykdinitkpasvfvqlrrksdlet sepkpflyypeikdkeevqrkrqkgssgtsgggsgggmilkilneiasi gstkqkqaileknkdnellkrvyrltysrglqyyikkwpkpgiatqsfg mltltdmldfieftlatrkltgnaaieeltgyitdgkkddvevlrrvmm rdlecgasvsiankvwpglipeqpqmlassydekginknikfpafaqlk adgarcfaevrgdelddvrllsragneylgldllkeelikmtaearqih pegvlidgelvyheqvkkepegldflfdaypenskakefaevaesrtas ngianksslkgtisekeaqcmkfqvwdyvplveiyslpafrlkydvrfsk leqmtsgydkvilienqvvnnldeakviykkyidqglegiilknidglw enarsknlykfkevidvdlkivgiyphrkdptkaggfilesecgkikvn agsglkdkagvksheldrtrimenqnyyigkilecencngwlksdgrtdy vklflpiairlredktkantfedvfgdfhevtgl | 17 |
| 4-7-5-9 | Mghhhhhhhhhssghiegrassptsymspslpaldwqlpshsgpyelr ievqpkshhrahyetegsrgavkasagghpivqlhgylenepltlqlfi gtaddrllrphafyqvhritgktvsttsheiilsntkvleipllpennm raiidcagilklrnsdielrkgetdigrkntrvrlvfrvhipqpngrtl slqasnlkivrmdrtagcvtggeeiyllcdkvqkddiqirfyeeeengg vwegfgdfsptdvhrqfaivfktpkykdinitkpasvfvqlrrksdlet sepkpflyypeikdkeevqrkrqkgssgtsgggsgggmrylelaqlyqk lekttmkliktrlvadflkkvpddhlefipylilgdvfpewderelgvg ekllikavamatgidaneiensvkdtgdlgesialavkkrkqksffsqp ltikrvyqtlvkvaettgegsqekkmkylanlfmdaepieakyiartvl gtmrtgvaegllrdaialafhvkvelveraymltsdfgfvakvaklegn eglakvqvqigkpikpmlaqqaanikeallemggeaefeikydgarvqv hkdgdkiivysrrlenvtraipeivealkqsvkpnkaivegelvaiged grplpfqyvlrrfrrkhniqemmkkiplelnlfdvlyvdgesmidvkfi drrkkleeiiepngkikvaenlitkkveeaeafykkalemgheglmakr ldatyepgnrgkkwlkikptmenldlviigaewgegrrahllgsfilga ydpetgeflevgkvgsgftdedlveftkmlkpliikeegkrvwiepkiv ievtyqeiqkspkyksgfalrfpryvalrddkgpedadtieriaqlyel qermkgkv | 18 |
| 4-7-5-10 | Mghhhhhhhhhhssghiegrassptsymspslpaldwqlpshsgpyelr ievqpkshhrahyetegsrgavkasagghpivqlhgylenepltlqlfi gtaddrllrphafyqvhritgktvsttsheiilsntkvleipllpennm raiidcagilklrnsdielrkgetdigrkntrvrlvfrvhipqpngrtl slqasnlkivrmdrtagcvtggeeiyllcdkvqkddiqirfyeeeengg vwegfgdfsptdvhrqfaivfktpkykdinitkpasvfvqlrrksdlet sepkpflyypeikdkeevqrkrqkgssgtsgggsgggmrylelaqlyqk lekttmkliktrlvadflkkvpddhlefipylilgdvfpewderelgvg ekllikavamatgidaneiensvkdtgdlgesialavkkrkqksffsqp ltikrvyqtlvkvaettgegsqekkmkylanlfmdaepieakyiartvl gtmrtgvaegllrdaialafhvkvelveraymltsdfgfvakvaklegn eglakvqvqigkpikpmlaqqaanikeallemggeaefeikydgarvqv hkdgdkiivysrrlenvtraipeivealkqsvkpnkaivegelvaiged grplpfqyvlrrfrrkhniqemmkkiplelnlfdvlyvdgesmidvkfi drrkkleeiiepngkikvaenlitkkveeaeafykkalemgheglmakr ldatyepgnrgkkwlkikptmenldlviigaewgegrrahllgsfilga ydpetgeflevgkvgsgftdedlveftkmlkpliikeegkrvwiepkiv | 19 |
| | ievtyqeiqkspkyksgfalrfpryvalrddkgp | |

The above-indicated fusion protein sequences provide examples of blunt-end ligases including DNA-ligase portions. Further fusion proteins believed to be useful as blunt-end ligases, and believed to be useful as thermostable nucleic acid blunt-end ligases, include further fusion proteins comprising variants of the fusion protein sequences described herein by SEQ ID NO:. In embodiments, such further fusion proteins believed to be useful as blunt-end ligases, and believed to be useful as thermostable nucleic acid blunt-end ligases, are homologous variants variants of the fusion protein sequences described herein by SEQ ID NO:, e.g., homologous variants having greater than 90%, or greater than 95%, or greater than 99% sequence homology or sequence identity to a fusion protein sequence described herein by SEQ ID NO:.

As discussed above, a second amino acid sequence having sequence homology to a first amino acid sequence may differ from the first amino acid by only conservative substitutions; that is, a residue in the second amino acid sequence is either a) identical to the corresponding residue in the first amino acid sequence; b) a member of the same group of amino acids as the corresponding residue in the first amino acid sequence, where the group is based on common side-chain properties as disclosed above: or c) an exemplary or preferred substitution (as identified above in Table 1C above).

For example, suitable fusion proteins include fusion proteins having greater than 90 %, or greater than 95% or greater than 99%, sequence homology or sequence identity to a fusion protein having a composition 4-DNA binding protein-5-DNA ligase (written in the N-terminal to C-terminal orientation), where "DNA binding protein" indicates the amino acid sequence of a DNA binding protein and "DNA ligase" indicates the amino acid sequence of a DNA ligase included in the fusion protein. As discussed above, examples of DNA binding proteins include SEQ ID NO: 2 and SEQ ID NO: 7; examples of DNA ligases include SEQ ID NO: 3, SEQ ID NO: 8, SEQ ID NO: 9, and SEQ ID NO: 10.

### EXAMPLE 3

Further fusion proteins believed to be useful as blunt-end ligases, and believed to be useful as thermostable nucleic acid blunt-end ligases, include fusion proteins as described herein, where the DNA-binding protein is replaced by a RNA-binding protein, and where the ligase comprises an RNA-ligase protein in fusion proteins having a composition 4-RNA binding protein-5-RNA ligase (written in the N-terminal to C-terminal orientation), where "RNA binding protein" indicates the amino acid sequence of a RNA binding protein and "RNA ligase" indicates the amino acid sequence of a RNA ligase included in the fusion protein. In embodiments, the RNA ligase may be a thermostable RNA ligase. Thus, a fusion protein as disclosed herein having RNA ligase activity, and believed to have thermostable RNA ligase activity, comprises a linear polypeptide having a composition including the covalently linked amino acid sequences (in the following N-terminal to C-terminal order) SEQ ID NO: 4, RNA binding protein, SEQ ID NO: 5, RNA ligase.

In addition, suitable RNA ligase fusion proteins include RNA ligase fusion proteins having greater than 90 %, or greater than 95% or greater than 99%, sequence homology to a fusion protein having a composition 4-RNA binding protein-5-RNA ligase. As discussed above, a second amino acid sequence having sequence homology to a first amino acid sequence may differ from the first amino acid by only conservative substitutions; that is, a residue in the second amino acid sequence is either a) identical to the corresponding residue in the first amino acid sequence; b) a member of the same group of amino acids as the corresponding residue in the first amino acid sequence, where the group is based on common side-chain properties as disclosed above: or c) an exemplary or preferred substitution (as identified above in Table 1C above).

### EXAMPLE 4

Proteins having features as disclosed herein include proteins that are homologous to any of the fusion proteins disclosed herein. Such proteins are believed to be suitable for use as nucleic acid ligases, including as thermostable nucleic acid ligases. For example, such proteins are believed to be suitable for use as blunt-end DNA ligases, including as thermostable blunt-end DNA ligases. Such proteins are further believed to be suitable for use as blunt-end RNA ligases, including as thermostable blunt-end RNA ligases.

Proteins that are homologous to any of the fusion proteins disclosed herein may be provided by providing variant proteins having greater than 90 %, or greater than 95% or greater than 99%, sequence homology to a fusion protein disclosed herein. For example, particular proteins that are homologous to any of the fusion proteins disclosed herein may be provided by providing variant proteins having greater than 90 %, or greater than 95% or greater than 99%, sequence homology or sequence identity to SEQ ID NO: 1, SEQ ID NO: 13, SEQ ID NO: 14, SEQ ID NO: 15, SEQ ID NO: 16, SEQ ID NO: 17, SEQ ID NO: 18, or SEQ ID NO: 19 (i.e., having greater than 90 %, or greater than 95% or greater than 99%, sequence homology or sequence identity to a fusion protein of Table 4).

Fusion proteins as disclosed herein include a composition having the general form of a linear molecule having a histidine-rich leader sequence covalently linked to a nucleic acid binding protein sequence which is covalently linked to a flexible glycine-rich sequence which is covalently linked to a nucleic acid ligase sequence. In embodiments, such fusion proteins may be proteins including one or more amino acid substitutions, insertions, deletions, or additions as compared to a fusion protein sequence provided as a sequence listing herein. In embodiments, such fusion proteins may be proteins including one or more amino acid substitutions, insertions, deletions, or additions as compared to a histidine-rich leader sequence, a nucleic acid binding protein sequence, a flexible glycine-rich sequence, or a nucleic acid ligase sequence provided as a sequence listing herein.

For example, homologous fusion proteins having features disclosed herein may include histidine-rich leader sequences different than, while homologous to, SEQ ID NO: 4; e.g., having histidine-rich leader sequences having greater than 90 %, or greater than 95% or greater than 99%, sequence homology or sequence identity to SEQ ID NO: 4. It will be understood that such homologous histidine-rich leader sequences may include the same number, or may include a greater number, or may include a smaller number, of amino acid residues than SEQ ID NO: 4.

For example, homologous fusion proteins having features disclosed herein may include flexible glycine-rich sequences different than, while homologous to, SEQ ID NO: 5; e.g., having flexible glycine-rich sequences having greater than 90 %, or greater than 95% or greater than 99%, sequence homology or sequence identity to SEQ ID NO: 5. It will be understood that such homologous flexible glycine-rich sequences may include the same number, or may include a greater number, or may include a smaller number, of amino acid residues than SEQ ID NO: 5.

For example, homologous fusion proteins include fusion proteins having compositions homologous to any of the proteins described herein as 4-2-5-3, 4-2-5-8, 4-2-5-9, and 4-2-5-10, where the sequence "2" is replaced by a sequence having greater than 90 %, or greater than 95% or greater than 99%, sequence homology or sequence identity to SEQ ID NO: 2; or where the sequence "3", or "8", or "9", or "10" is replaced by a sequence having greater than 90 %, or greater than 95% or greater than 99%, sequence homology or sequence identity to SEQ ID NO: 3, SEQ ID NO: 8, SEQ ID NO: 9, or SEQ ID NO: 10. In embodiments, both the sequence "2" is replaced by a homologous sequence and the sequence "3", or "8", or "9", or "10" is replaced by a homologous sequence.

In addition, homologous fusion proteins include fusion proteins having compositions homologous to any of the proteins described herein as 4-7-5-3, 4-7-5-8, 4-7-5-9, and 4-7-5-10, where the sequence "7" is replaced by a sequence having greater than 90 %, or greater than 95% or greater than 99%, sequence homology or sequence identity to SEQ ID NO: 7; or where the sequence "3", or "8", or "9", or "10" is replaced by a sequence having greater than 90 %, or greater than 95% or greater than 99%, sequence homology or sequence identity to SEQ ID NO: 3, SEQ ID NO: 8, SEQ ID NO: 9, or SEQ ID NO: 10. In embodiments, both the sequence "7" is replaced by a homologous sequence and the sequence "3", or "8", or "9", or "10" is replaced by a homologous sequence.

While the above is a complete description of the preferred embodiment as described herein, it is possible to use various alternatives, modifications and equivalents. Therefore, the scope of the present invention should be determined not with reference to the above description but should, instead, be determined with reference to the appended claims, along with their full scope of equivalents. Any feature, whether preferred or not, may be combined with any other feature, whether preferred or not. The appended claims are not to be interpreted as including means-plus-function limitations, unless such a limitation is explicitly recited in a given claim using the phrase "means for." It should be understood that as used in the description herein and throughout the claims that follow, the meaning of "a," "an," and "the" includes plural reference unless the context clearly dictates otherwise. Also, as used in the description herein and throughout the claims that follow, the meaning of "in" includes "in" and "on" unless the context clearly dictates otherwise. Finally, as used in the description herein and throughout the claims that follow, the meanings of "and" and "or" include both the conjunctive and disjunctive and may be used interchangeably unless the context expressly dictates otherwise. Thus, in contexts where the terms "and" or "or" are used, usage of such conjunctions do not exclude an "and/or" meaning unless the context expressly dictates otherwise.

## Claims

1. A thermostable blunt-end DNA ligase comprising a fusion protein comprising, in N-terminal to C-terminal order, a leader sequence comprising the amino acid sequence SEQ ID NO: 4, a DNA binding protein, a flexible glycine-rich amino acid sequence, and a DNA ligase, wherein said thermostable blunt-end DNA ligase is suitable for use in a blunt-ended DNA ligation reaction performed at 60 °C or higher.

2. The thermostable blunt-end DNA ligase of claim 1, comprising a T4 DNA ligase with an N-terminal p50 fusion.

3. The thermostable blunt-end DNA ligase of claim 1, comprising an amino acid sequence selected from the group of amino acid sequences consisting of SEQ ID NO: 1 , SEQ ID NO: 13, SEQ ID NO: 14, SEQ ID NO: 15, SEQ ID NO: 16, SEQ ID NO: 17, SEQ ID NO: 18, and SEQ ID NO: 19.

4. A method of ligating blunt-end DNA at a temperature of 60°C or higher, comprising ligating blunt-end DNA using a thermostable blunt-end DNA ligase suitable for use in a blunt-ended DNA ligation reaction performed at 60 °C or higher, wherein said thermostable blunt-end DNA ligase comprises a thermostable blunt-end DNA ligase of claim 1.

5. An article of manufacture, comprising a thermostable blunt-end DNA ligase suitable for use in a blunt-ended DNA ligation reaction performed at 60 °C or higher and a container, wherein said thermostable blunt-end DNA ligase comprises a thermostable blunt-end DNA ligase of claim 1.

6. The article of manufacture of claim 5, further comprising a buffer.

7. A device for analyzing a sample containing DNA, wherein the device comprises a thermostable blunt-end DNA ligase suitable for use in a blunt-ended DNA ligation reaction performed at 60 °C or higher, wherein said thermostable blunt-end DNA ligase is selected from a thermostable blunt-end DNA ligase of claim 1.

8. The method of claim 4, wherein said thermostable blunt-end DNA ligase is suitable for use in a blunt-ended DNA ligation reaction performed at 75 °C, and said ligating is performed at 75 °C.

9. The thermostable blunt-end DNA ligase of claim 1, wherein said thermostable blunt-end DNA ligase is capable of making concatamers upon multiple ligation events in a blunt-ended DNA ligation reaction performed at 60 °C or higher.

10. The thermostable blunt-end DNA ligase of claim 1, wherein said thermostable blunt-end DNA ligase is suitable for use in a nucleic acid amplification scheme which operates at a uniform temperature of 60 °C or higher.

11. The thermostable blunt-end DNA ligase of claim 1, wherein said fusion protein comprises a component selected from a peptide linker, an N-terminal addition, a C-terminal addition, a tag peptide, a D-amino acid, a peptide mimetic, a sugar and a polymer.

12. The thermostable blunt-end DNA ligase of claim 1, wherein said flexible glycine-rich amino acid sequence comprises SEQ ID NO: 5.

13. A method of ligating blunt-end nucleic acids at an elevated temperature, comprising using a thermostable blunt-end nucleic acid ligase of claim 3 at a temperature of 60 °C or higher.

14. A device for analyzing a sample containing nucleic acids, wherein the device comprises a thermostable blunt-end nucleic acid ligase of claim 1.

15. The device of claim 14, wherein said thermostable blunt-end ligase comprises an amino acid sequence selected from SEQ ID NO: 1, SEQ ID NO: 13, SEQ ID NO: 14, SEQ ID NO: 15, SEQ ID NO: 16, SEQ ID NO: 17, SEQ ID NO: 18, and SEQ ID NO: 19.

## Patentansprüche

1. Thermostabile stumpfendige DNA-Ligase, umfassend ein Fusionsprotein, das in einer Reihenfolge von N-Terminus zu C-Terminus eine die Aminosäuresequenz SEQ ID NO: 4 umfassende Leitsequenz, ein DNA-bindendes Protein, eine flexible glycinreiche Aminosäuresequenz und eine DNA-Ligase umfasst, wobei die thermostabile stumpfendige DNA-Ligase zur Verwendung in einer bei mindestens 60 °C durchgeführten Ligationsreaktion von stumpfendiger DNA geeignet ist.

2. Thermostabile stumpfendige DNA-Ligase nach Anspruch 1, umfassend eine T4-DNA-Ligase mit einer N-terminalen p50-Fusion.

3. Thermostabile stumpfendige DNA-Ligase nach Anspruch 1, umfassend eine Aminosäuresequenz, ausgewählt aus der Gruppe von Aminosäuresequenzen bestehend aus SEQ ID NO: 1, SEQ ID NO: 13, SEQ ID NO: 14, SEQ ID NO: 15, SEQ ID NO: 16, SEQ ID NO: 17, SEQ ID NO: 18 und SEQ ID NO: 19.

4. Verfahren zum Ligieren einer stumpfendigen DNA bei einer Temperatur von mindestens 60 °C, umfassend das Ligieren einer stumpfendigen DNA unter Verwendung einer thermostabilen stumpfendigen DNA-Ligase, die zur Verwendung in einer bei mindestens 60 °C durchgeführten Ligationsreaktion von stumpfendiger DNA geeignet ist, wobei die thermostabile stumpfendige DNA-Ligase eine thermostabile stumpfendige DNA-Ligase nach Anspruch 1 umfasst.

5. Herstellungserzeugnis, umfassend eine thermostabile stumpfendige DNA-Ligase, die zur Verwendung in einer bei mindestens 60 °C durchgeführten Ligationsreaktion von stumpfendiger DNA geeignet ist, und einen Behälter, wobei die thermostabile stumpfendige DNA-Ligase eine thermostabile stumpfendige DNA-Ligase nach Anspruch 1 umfasst.

6. Herstellungserzeugnis nach Anspruch 5, ferner einen Puffer umfassend.

7. Vorrichtung zum Analysieren einer DNA-haltigen Probe, wobei die Vorrichtung eine thermostabile stumpfendige DNA-Ligase, die zur Verwendung in einer bei mindestens 60 °C durchgeführten Ligationsreaktion von stumpfendiger DNA geeignet ist, umfasst, wobei die thermostabile stumpfendige DNA-Ligase aus einer thermostabilen stumpfendigen DNA-Ligase nach Anspruch 1 ausgewählt ist.

8. Verfahren nach Anspruch 4, wobei die thermostabile stumpfendige DNA-Ligase zur Verwendung in einer bei 75 °C durchgeführten Ligationsreaktion von stumpfendiger DNA geeignet ist und das Ligieren bei 75 °C durchgeführt wird.

9. Thermostabile stumpfendige DNA-Ligase nach Anspruch 1, wobei die thermostabile stumpfendige DNA-Ligase in der Lage ist, nach mehreren Ligationsereignissen in einer bei mindestens 60 °C durchgeführten Ligationsreaktion von stumpfendiger DNA Konkatamere herzustellen.

10. Thermostabile stumpfendige DNA-Ligase nach Anspruch 1, wobei die thermostabile stumpfendige DNA-Ligase zur Verwendung in einem Nukleinsäureamplifikationsschema, das bei einer einheitlichen Temperatur von mindestens 60 °C wirkt, geeignet ist.

11. Thermostabile stumpfendige DNA-Ligase nach Anspruch 1, wobei das Fusionsprotein eine Komponente umfasst, die ausgewählt ist aus einem Peptidlinker, einer N-terminalen Addition, einer C-terminalen Addition, einem Markierungspeptid, einer D-Aminosäure, einem Peptidmimetikum, einem Zucker und einem Polymer.

12. Thermostabile stumpfendige DNA-Ligase nach Anspruch 1, wobei die flexible glycinreiche Aminosäuresequenz SEQ ID NO: 5 umfasst.

13. Verfahren zum Ligieren von stumpfendigen Nukleinsäuren bei einer erhöhten Temperatur, umfassend das Verwenden einer thermostabilen stumpfendigen Nukleinsäureligase nach Anspruch 3 bei einer Temperatur von mindestens 60 °C.

14. Vorrichtung zum Analysieren einer Probe, die Nukleinsäuren enthält, wobei die Vorrichtung eine thermostabile stumpfendige Nukleinsäureligase nach Anspruch 1 umfasst.

15. Vorrichtung nach Anspruch 14, wobei die thermostabile stumpfendige Ligase eine Aminosäuresequenz umfasst, die ausgewählt ist aus SEQ ID NO: 1, SEQ ID NO: 13, SEQ ID NO: 14, SEQ ID NO: 15, SEQ ID NO: 16, SEQ ID NO: 17, SEQ ID NO: 18 und SEQ ID NO: 19.

## Revendications

1. ADN ligase à extrémités franches thermostable comprenant une protéine de fusion comprenant, de l'extrémité N-terminale à l'extrémité C-terminale, une séquence de tête comprenant la séquence d'acides aminés SEQ ID n° : 4, une protéine de liaison à l'ADN, une séquence d'acides aminés flexible riche en glycine et une ADN ligase, où ladite ADN ligase à extrémités franches thermostable est appropriée pour une utilisation dans une réaction de ligature d'ADN à extrémités franches effectuée à 60 °C ou plus.

2. ADN ligase à extrémités franches thermostable selon la revendication 1, comprenant une ADN ligase T4 avec une fusion p50 N-terminale.

3. ADN ligase à extrémités franches thermostable selon la revendication 1, comprenant une séquence d'acides aminés choisie dans le groupe de séquences d'acides aminés constituées par SEQ ID n° : 1, SEQ ID n° : 13, SEQ ID n° : 14, SEQ ID n° : 15, SEQ ID n° : 16, SEQ ID n° : 17, SEQ ID n° : 18 et SEQ ID n° : 19.

4. Procédé de ligature d'ADN à extrémités franches à une température de 60 °C ou plus, comprenant la ligature d'ADN à extrémités franches à l'aide d'une ADN ligase à extrémités franches thermostable appropriée pour une utilisation dans une réaction de ligature d'ADN à extrémités franches effectuée à 60 °C ou plus, où ladite ADN ligase à extrémités franches thermostable comprend une ADN ligase à extrémités franches thermostable selon la revendication 1.

5. Article manufacturé, comprenant une ADN ligase à extrémités franches thermostable appropriée pour une utilisation dans une réaction de ligature d'ADN à extrémités franches effectuée à 60 °C ou plus et un récipient, où ladite ADN ligase à extrémités franches thermostable comprend une ADN ligase à extrémités franches thermostable selon la revendication 1.

6. Article manufacturé selon la revendication 5, comprenant en outre un tampon.

7. Dispositif d'analyse d'un échantillon contenant de l'ADN, lequel dispositif comprend une ADN ligase à extrémités franches thermostable appropriée pour une utilisation dans une réaction de ligature d'ADN à extrémités franches effectuée à 60 °C ou plus, où ladite ADN ligase à extrémités franches thermostable comprend une ADN ligase à extrémités franches thermostable selon la revendication 1.

8. Procédé selon la revendication 4, où ladite ADN ligase à extrémités franches thermostable est appropriée pour une utilisation dans une réaction de ligature d'ADN à extrémités franches effectuée à 75 °C, et ladite ligature est effectuée à 75 °C.

9. ADN ligase à extrémités franches thermostable selon la revendication 1, laquelle ADN ligase à extrémités franches thermostable est capable de produire des concatémères lors de multiples événements de ligature dans une réaction de ligature d'ADN ligase à extrémités franches effectuée à 60 °C ou plus.

10. ADN ligase à extrémités franches thermostable selon la revendication 1, laquelle ADN ligase à extrémités franches thermostable est appropriée pour une utilisation dans un programme d'amplification d'acides nucléiques qui se déroule à une température uniforme de 60 °C ou plus.

11. ADN ligase à extrémités franches thermostable selon la revendication 1, où ladite protéine de fusion comprend un composant choisi parmi un lieur peptidique, une addition N-terminale, une addition C-terminale, un peptide marqueur, un acide aminé D, un mimétique peptidique, un sucre et un polymère.

12. ADN ligase à extrémités franches thermostable selon la revendication 1, où ladite séquence d'acides aminés flexible riche en glycine comprend SEQ ID n° : 5.

13. Procédé de ligature d'acides nucléiques à extrémités franches à une température élevée, comprenant l'utilisation d'une acide nucléique ligase à extrémités franches selon la revendication 3 à une température de 60 °C ou plus.

14. Dispositif d'analyse d'un échantillon contenant des acides nucléiques, lequel dispositif comprend une acide nucléique ligase à extrémités franches thermostable selon la revendication 1.

15. Dispositif selon la revendication 14, où ladite ligase à extrémités franches thermostable comprend une séquence d'acides aminés choisie parmi SEQ ID n° : 1, SEQ ID n° : 13, SEQ ID n° : 14, SEQ ID n° : 15, SEQ ID n° : 16, SEQ ID n° : 17, SEQ ID n° : 18 et SEQ ID n° : 19.
